# EUROPEAN PATENT APPLICATION

(11) **EP 4 289 289 A2**
(43) Date of publication of application: **13.12.2023**
(21) Application number: 23206116.8
(22) Date of filing: 01.05.2020
(51) Int. Cl.: A23P 20/18

(54) **SYSTEMS AND METHODS FOR MANUFACTURING CANNABIS EDIBLES, AND RESULTING EDIBLE PRODUCTS**

(30) Priority: 06.05.2019 US 201962844060 P; 21.10.2019 US 201962923668 P; 09.04.2020 US 202063007881 P
(62) Divisional of application: 20731620.9
(71) Applicant: Transport Authority, Inc., Dover, DE 19901 (US)
(72) Inventor: WARREN, William, Dover, DE, 19901 (US); DOYLE, David, Dover, DE, 19901 (US)
(74) Representative: Bardehle Pagenberg Partnerschaft mbB Patentanwälte Rechtsanwälte

(57) **Abstract**

The present disclosure is directed to a method of quantifying an active pharmaceutical ingredient delivered to a food product. The method comprises the following steps: (i) Causing a plurality of microdroplets to travel out of a dosing head of a dosing system toward the food product, wherein the microdroplets containing the active pharmaceutical ingredient that includes a cannabinoid. (ii) Measuring a cross-sectional dimension of each of the microdroplets with a camera as the microdroplets travel from the dosing head to the food product. (iii) Based on the measuring step, calculating an approximation of a volume of the microdroplets delivered to the food product.

## Description

### CROSS-REFERENCE TO RELATED APPLICATIONS

This claims priority to U.S. Patent Application Serial No. 62/844,060 filed May 6, 2019, U.S. Patent Application Serial No. 62/923,668 filed October 21, 2019, and U.S. Patent Application Serial No. 63/007,881 filed April 9, 2020, the disclosure of each of which is hereby incorporated by reference as if set forth in its entirety herein.

### BACKGROUND

### Field of the Art

The disclosure relates to the field of cannabis products, and more particularly to the field of edible products comprising cannabis, and methods and systems for manufacturing the same.

### Discussion of the State of the Art

The legal cannabis industry is growing rapidly in the United States, Canada, and across the world. However, rules and regulations at the local, state, and country level vary dramatically from location to location and are changing rapidly. This is especially true for edible cannabis products that must comply with both food related rules and regulations as well as cannabis rules and regulations. Issues include contamination and dosing testing, seed-to-sale tracking, and costly cannabis taxes. What's legal or allowable in one location is sometimes not legal or allowable in another, creating a barrier for the transportation of finished edible cannabis products from one region to another.

Compounding these issues is that the processes used to manufacture edible cannabis products have been designed without regard to these complex rules and regulations. Cannabis has been treated as any other base ingredient in these food products and has simply been added with the others (importantly, food manufacturing has evolved without any thought to controlled substances; only basic cleanliness standards are required). As manufacturers try to scale their operations, it becomes difficult for them to ensure the exact does in each piece. In addition, cooking and baking with heat degrades cannabis, reducing the effectiveness of the finished product. Often cannabis-related taxes may be stacked as a product moves between jurisdictions. In many cases, movement of cannabis-based products between jurisdictions is illegal and possibly even felonious, and can carry tax penalties. For example, while manufacturing and selling cannabis edibles in California is legal *in California,* transport of such products across the state line constitutes a felony under Federal law. Different nations, states, municipalities, and regions within municipalities also can have regulations that govern the transport of such products across their respective jurisdictional boundaries. These issues tend to squeeze margins for cannabis-based edibles, particularly by in effect forcing local manufacturing in each jurisdiction, eliminating or minimizing manufacturing economies of scale.

Therefore, edibles products have needed to be manufactured in the general area of the rules and regulations. This effectively requires building a new food manufacturing industry that specializes on cannabis edible products and then replicating it state-by-state, or region-by-region. This makes it difficult to scale and match product quality from factory to factory and it limits small batch flexibility. As a result, it is very expensive and complex to roll out national and international brands.

What is clearly needed is a system and method for manufacturing cannabis edibles, and resulting edible products where the based edible food products are manufactured normally at centralized facilities enjoying economies of scale, and cannabis plant extract is added in a second stage, locally to the intended vending and consumption of the final product.
Reference document US 2019/0015383 A1 relates to cannabinoid compositions and methods of preparation and use thereof, e.g., in foods and beverages.

### SUMMARY

Accordingly, methods for manufacturing edibles and non-edibles containing at least one API are provided. The at least one active pharmaceutical ingredient can include one or more of cannabis or a cannabinoid, at least one flavonoid, at least one terpene, an over the counter (OTC) drug and a prescription drug.

In one aspect, a method of delivering an active pharmaceutical ingredient (API) to a substrate. The method can include the step of bringing the substrate into alignment with a dosing station that includes a dosing head. The method can further include the step of after the bringing step, delivering at least one microdroplet of an API-containing liquid from the dosing head to the substrate, wherein the at least one microdroplet contains a predetermined approximate quantity of API.

In one aspect, the method adds cannabis to finished or nearly-finished food products. This allows the base food product to be manufactured in one location and transported across a border to another location where the cannabis is then added. Therefore, conventional food manufacturers can be used as outsource suppliers to the cannabis industry, leveraging their factories, expertise, and economies of scale. The cannabis component added later is not subjected to the cooking and baking of the base food product and therefore does not degrade. Economic efficiency, small batch flexibility, and improved quality and consistency are all benefits of our new method. As a result, national and international brands can be rolled out across borders with substantially lower capital equipment requirements, lower real estate costs, providing the ability to scale faster to large volumes.

According to various aspects, further benefits may include extremely accurate dosing; boxes and labeling may be made before manufacturing, and in volume; much lower scrap rate than previously achievable are possible; and far lower sampling plans are feasible because the bulk product is manufactured in a predictable, unregulated way and only the final step, such as the addition of a cannabinoid, needs to be tested. Another benefit is the feasibility of manufacturing extremely small runs, for specialty products or extremely small markets, as the bulk of the manufacturing is accomplished in a centralized, highly-scalable way and only inexpensive addition of at least one cannabinoid is done locally. Thus, products can be tailored to finely-segmented markets or very small local regulatory regimes. Even personalized formulations may be delivered, according to the invention, for example to provide accurate personal dosing for medicinal cannabis in a scalably manufactured food product. Another benefit of the above aspects is that it is possible to react very quickly to changes in markets-whether regulatory or demand-by taking advantage of the just-in-time addition of the at least one cannabinoid.

In one aspect, a system is provided for delivering an active pharmaceutical ingredient to a prepared substrate. The system can include a delivery station that includes a support surface configured to receive the prepared substrate. The system can further include a dosing station that is configured to deliver to the substrate a predetermined approximate dose of the active pharmaceutical ingredient. At least one of the support surface and the dosing station is movable with respect to the other of the support surface and the dosing station so as to align the prepared substrate with the dosing station after the support surface has received the prepared substrate.

### BRIEF DESCRIPTION OF THE DRAWINGS

The following detailed description will be better understood when read in conjunction with the appended drawings, in which there is shown in the drawings example embodiments for the purposes of illustration. It should be understood, however, that the present disclosure is not limited to the precise arrangements and instrumentalities shown. In the drawings:
Fig. 1A is a schematic perspective view of a system for delivering an active pharmaceutical ingredient to an edible product;
Fig. 1B is an enlarged schematic perspective view of a portion of the edible product illustrated in Fig. 1A;
Fig. 2A is a schematic view of a dosing zone of the system illustrated in Fig. 1A;
Fig. 2B is a perspective view of a microdroplet in one example;
Fig. 2C is a side elevation view of a microdroplet in another example;
Fig. 3 is a plan view of the edible product illustrated in Fig. 1A, showing a delivery zone;
Fig. 4A is a perspective view of mixed nuts dosed with an active pharmaceutical ingredient in accordance with aspects of the present disclosure;
Fig. 4B is a perspective view of dried fruit dosed with an active pharmaceutical ingredient in accordance with aspects of the present disclosure;
Fig. 4C is a perspective view of a baked good dosed with an active pharmaceutical ingredient in accordance with aspects of the present disclosure;
Fig. 4D is a side elevation view of a gummy candy dosed with an active pharmaceutical ingredient in accordance with aspects of the present disclosure;
Fig. 4E is a perspective view of a tongue depressor dosed with an active pharmaceutical ingredient in accordance with aspects of the present disclosure;
Fig. 5A is a perspective view of a dosing machine constructed in accordance with one example; and
Fig. 5B is a schematic view of a method of dosing using the dosing machine illustrated in Fig. 5A.

### DETAILED DESCRIPTION

Disclosed are systems and methods for manufacturing cannabis edibles, and resulting edible products.

One or more different aspects may be described in the present application. Further, for one or more of the aspects described herein, numerous alternative arrangements may be described; it should be appreciated that these are presented for illustrative purposes only and are not limiting of the aspects contained herein or the claims presented herein in any way. One or more of the arrangements may be widely applicable to numerous aspects, as may be readily apparent from the disclosure. In general, arrangements are described in sufficient detail to enable those skilled in the art to practice one or more of the aspects, and it should be appreciated that other arrangements may be utilized and that structural, logical, software, electrical and other changes may be made without departing from the scope of the particular aspects. Particular features of one or more of the aspects described herein may be described with reference to one or more particular aspects or figures that form a part of the present disclosure, and in which are shown, by way of illustration, specific arrangements of one or more of the aspects. It should be appreciated, however, that such features are not limited to usage in the one or more particular aspects or figures with reference to which they are described. The present disclosure is neither a literal description of all arrangements of one or more of the aspects nor a listing of features of one or more of the aspects that must be present in all arrangements.

Headings of sections provided in this patent application and the title of this patent application are for convenience only and are not to be taken as limiting the disclosure in any way.

Devices that are in communication with each other need not be in continuous communication with each other, unless expressly specified otherwise. In addition, devices that are in communication with each other may communicate directly or indirectly through one or more communication means or intermediaries, logical or physical.

A description of an aspect with several components in communication with each other does not imply that all such components are required. To the contrary, a variety of optional components may be described to illustrate a wide variety of possible aspects and in order to more fully illustrate one or more aspects. Similarly, although process steps, method steps, algorithms or the like may be described in a sequential order, such processes, methods and algorithms may generally be configured to work in alternate orders, unless specifically stated to the contrary. In other words, any sequence or order of steps that may be described in this patent application does not, in and of itself, indicate a requirement that the steps be performed in that order. The steps of described processes may be performed in any order practical. Further, some steps may be performed simultaneously despite being described or implied as occurring non-simultaneously (e.g., because one step is described after the other step). Moreover, the illustration of a process by its depiction in a drawing does not imply that the illustrated process is exclusive of other variations and modifications thereto, does not imply that the illustrated process or any of its steps are necessary to one or more of the aspects, and does not imply that the illustrated process is preferred. Also, steps are generally described once per aspect, but this does not mean they must occur once, or that they may only occur once each time a process, method, or algorithm is carried out or executed. Some steps may be omitted in some aspects or some occurrences, or some steps may be executed more than once in a given aspect or occurrence.

When a single device or article is described herein, it will be readily apparent that more than one device or article may be used in place of a single device or article. Similarly, where more than one device or article is described herein, it will be readily apparent that a single device or article may be used in place of the more than one device or article.

The functionality or the features of a device may be alternatively embodied by one or more other devices that are not explicitly described as having such functionality or features. Thus, other aspects need not include the device itself.

Techniques and mechanisms described or referenced herein will sometimes be described in singular form for clarity. However, it should be appreciated that particular aspects may include multiple iterations of a technique or multiple instantiations of a mechanism unless noted otherwise. Process descriptions or blocks in figures should be understood as representing modules, segments, or portions of code which include one or more executable instructions for implementing specific logical functions or steps in the process. Alternate implementations are included within the scope of various aspects in which, for example, functions may be executed out of order from that shown or discussed, including substantially concurrently or in reverse order, depending on the functionality involved, as would be understood by those having ordinary skill in the art.

### Methods and Systems for Adding Cannabis to Edible products

According to an aspect, methods for transforming an edible products or other substrates into a cannabis-containing products are disclosed. The method can include the step of delivering the cannabinoid to the substrate, which can be an edible product or non-edible product. The delivering step can be performed with any suitable one or more applicators that deliver a predetermined dose of the cannabinoid. The term "cannabinoid" as used herein refers to any extract from a marijuana plant or hemp plant, such as CBD, THC, or any alternative cannabinoid, alone or in combination with any one or more of a flavonoid or terpene. The extract can be in its pure form or processed as desired. While this disclosure provides for the addition of at least one cannabinoid to a substrate, and thus cannabinoids are contemplated as a market for the final product, applications of the systems and methods disclosed herein are possible and envisioned beyond cannabis, including (but not limited to) other active pharmaceutical ingredients (API). For instance, applications of the systems and methods disclosed herein are possible and envisioned to include active pharmaceutical ingredients (APIs) including one or more cannabinoids, any alternative one or more OTC or prescription drugs including those that provide one or both of a health benefit or recreational drug experience, or otherwise controlled ingestible materials. Thus, reference herein to an active pharmaceutical ingredient can include any one the following: cannabis, one or more cannabinoids, one or more over-the-counter drugs, one or more prescription drugs, one or more flavonoids, and one or more terpenes. Similarly, reference to one or more of the active pharmaceutical ingredients identified above can apply equally to any other of the active pharmaceutical ingredients identified above. According to an aspect of the present disclosure, a method of delivering a cannabinoid or conventional drug may also be used to deliver homeopathic remedies, herbal supplements with flavors or odors, and so forth to an edible product. The resulting edible product can be referred to as a "nutraceutical," as its definition is "a food containing health-giving additives or having medicinal benefit."

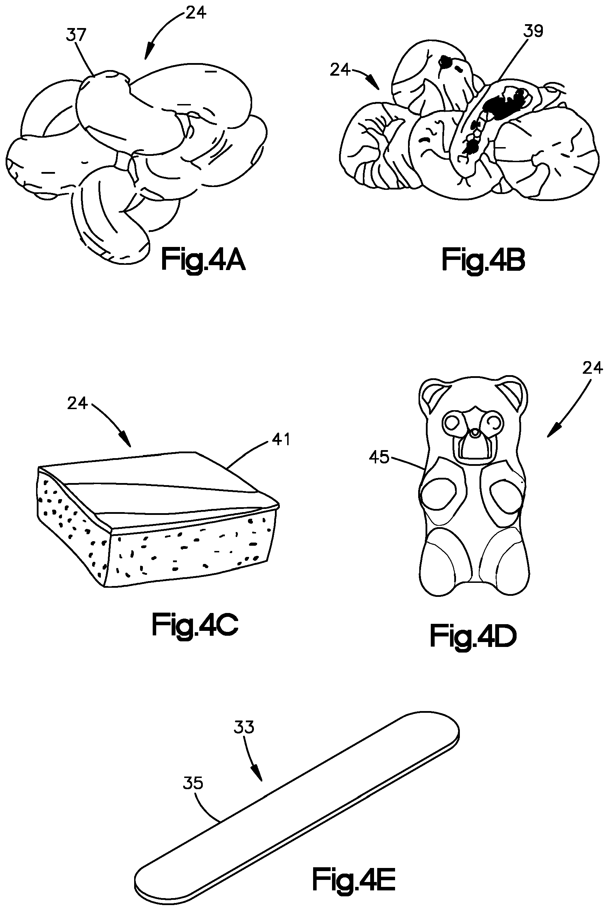

The active pharmaceutical ingredient can be added to a substrate to produce an active-containing substrate, which can include a prepared edible products or other inedible substrates. Prepared edible products can include, by way of example and not limitation, prepared food product such as hard candy, chocolates brownies, cookies, soft candies such as gummy candy, savories such as trail mix bars or dried meat pieces, and the like. Thus, in some examples, the prepared edible products can be cooked food product. In some specific examples, the prepared edible products can be baked food product. The prepared edible product can be bite sized, such as M&M candy, gummy candies, chocolate kisses, or the like, or can be designed to require more than one bite for full consumption, such as a cookie. Thus, it should be appreciated that the prepared edible product can be a cooked food product. For instance, the prepared edible product can be a baked food product. Thus, in some examples, the prepared edible product can include a plurality of mixed ingredients. In some examples, the prepared edible products can be a dehydrated food product, such as dried fruit or jerky. In other examples, the prepared edible product can be freeze dried. In still other examples, the prepared edible product can be a raw food product, such as nuts or fruit. It will thus be appreciated that the application of the active pharmaceutical ingredient to the previously prepared food product can allow for a broader range of food product to be made with active pharmaceutical ingredients. It will be appreciated that because the active pharmaceutical ingredients are applied to previously prepared food product, active pharmaceutical ingredients having short shelf lives can be applied to the substrate and ingested in a shorter period of time with respect to active pharmaceutical ingredients that are combined with the raw ingredients that are then processed to prepare the food product. Alternatively still, as noted above, the substrate can be an inedible product 33. That is, the substrate is not designed for human consumption, but is designed to be placed into the mouth. One such nonlimiting example is a tongue depressor 35 (see Fig. 4E).

In one aspect, a plurality of active-containing substrates can be provided as a set, wherein some of the substrates have different dosages of the active pharmaceutical ingredients and are designed to be ingested at different times among a period of time, such as different days of the week. Thus, a desired dosage profile can be delivered to the patient throughout the period of time. Alternatively or additionally, one of the active-containing substrates can contain a different at least one active pharmaceutical ingredient. Thus, the set of active-containing substrates can be designed to be sequentially ingested (that is, ingested one after the other) over the period of time, thereby delivering a desired predetermined sequence of active pharmaceutical ingredients to the patient.

The active pharmaceutical ingredient, including one or more cannabinoids, can be delivered to the substrate in liquid form as an API-containing liquid or in granular solid form as an API-containing solid. The API-containing liquid can be in the form of a pure API, such as a resin, or can be in the form of a concentration of API in a liquid carrier such as a solvent. The API-containing solid can be in the form of pure API, such as a powder, or can be a mixture of the API with any other suitable material. API-containing liquids and API-containing powders to be delivered to a substrate can be referred to as API-containing material. In some examples, an applicator can deliver microquantity of the active pharmaceutical ingredient to the substrate. For instance, the microquantity can be carried by a solvent and delivered by the applicator as microdroplets each having a volume in a range from approximately 2 nanoliters to approximately 10 microliters, such as from approximately 25 nanoliters to approximately 2 microliters. For instance, the microdroplets can have a volume that is in a range from approximately 25 nanoliters to approximately 1 microliter. The microdroplets can have a concentration of API as desired. For instance, the concentration of API can range from approximately 50 micrograms per microliter of solution to approximately 1 milligram per microliter of solution. In other examples, the liquid can be a pure resin of the API.

It is envisioned in one example that applicators delivering like dosages of API to like substrates can deliver approximately the same sized microdroplets to the like substrates. Thus, for instance when delivering the API to dried fruits and/or nuts designed to have the same dosage of API, the applicators can deliver the approximately same volume and number of microdroplets to each dried fruit and/or nut, or to a group, such as a serving, of dried fruits and/or nuts.

As will be described in more detail below, dosing heads can be provided that are configured to deliver microdroplets of any suitable volume, such as the volumes described above. Therefore, each microdroplet can contain a microquantity of API in a range from approximately 0.1 micrograms to approximately 10 milligrams, such as from approximately 1 milligram to approximately 2 milligrams. It is recognized, however, that the microdroplets can have any volume as desired. Further, It is recognized that each microdroplet can contain different quantities of API depending, for instance, on the volume of the microdroplet. The microquantity of API in a microdroplet can allow the dosage of API delivered to a substrate to be precisely controlled. For instance, the respective volumes of microdroplets can be delivered to a substrate within a range from approximately 1% to approximately 10%, for instance approximately 5%, from a target volume of microdroplets at three sigma. Thus, the dosage of API in each microdroplet can be delivered to the substrate within a range from approximately 1% to approximately 10%, for instance approximately 5%, from a target dosage at three sigma. The target volume of microdroplets can vary based on the surface area or volume of the substrate to which the microdroplets are to be applied. Similarly, the dosage of API to be delivered to a substrate can likewise be within a range from approximately 1% to approximately 10%, for instance approximately 5% at three sigma, from a target dosage of API to the substrate or a serving of packaged substrates, such as dried fruits or nuts. The target volume of microdroplets can vary based on the surface area or volume of the substrate to which the microdroplets are to be applied.

Thus, in one example, the microquantity can be delivered to a substrate in the form of microdroplets. The use of microdroplets may help in precise dosage administration of the active pharmaceutical ingredient compared to conventional techniques. The microdroplets can be delivered to an outer surface of the substrate by a 3D printer, inkjet printer, or other suitable printing process. Alternatively or additionally, the microdroplets can be delivered to the outer surface of the substrate by precision spraying. Alternatively or additionally still, the microdroplets can be delivered to an internal location of the substrate that is surrounded by the outer surface. For instance, the microdroplets can be mechanically injected, or can be delivered with an air gun that shoots the microdroplets toward the substrate in a burst of high-pressure air (in an analogous way to how some vaccines and other drugs may be administered subcutaneously without injections with needles). Without being bound by theory, it is believed that the microdroplets to an internal location of the substrate may also assist in facilitating administration or ingestion of potentially bitter-tasting (or strongly cannabis-tasting) formulations by providing a means to add small amounts of such material to a much larger amount of edible product.

The microdroplets including the API can be delivered to the substrate. The microdroplets can include a solution that includes the at least one cannabinoid in its liquid form as the solvent mixed with any suitable solute. In order to assist in achieving predictable doses of the at least one cannabinoid, the at least one cannabinoid can be substantially homogeneously mixed with the solute. Alternatively, the microdroplets can consist of or consist essentially of the cannabinoid extract, either purified, partially purified, or unpurified, in liquid form having a desired viscosity that allows for the cannabinoid extract to be reliably dispensed. In some examples, the liquid can be heated to achieve the desired viscosity without mixing the cannabinoid extract in a solute. In some examples, the microdroplets can have an oily or hydrophilic nature. For instance, microdroplets may be multilayered, with a protein or other protective coating surrounding a precise dosage of an oil-based or water-based formulation. Because the concentration of the cannabinoid in the liquid can be known, a volume of liquid can be predetermined and delivered to the substrate to achieve a desired predetermined approximate dose of the cannabinoid.

In other aspects, the active pharmaceutical ingredient, which can include one or more cannabinoids, can be added to the substrate in a granular form. For instance, the cannabinoid extract, which can be purified, partially purified, or unpurified, can be delivered to the substrate as a powder. In some examples, the cannabinoid can be crystallized and ground to produce the powder. Because the concentration of the cannabinoid in the powder can be known, a mass of the powder can be predetermined and delivered to the substrate to achieve a desired dose of the cannabinoid.

The liquid or powder to be delivered to the substrate can include a single desired cannabinoid. Thus, the single desired cannabinoid can be delivered to the substrate. Alternatively, a plurality of different liquids or powders can be delivered to the substrate, each containing their own different one or more cannabinoids. Accordingly, by delivering multiple different liquids and powders, a plurality of desired cannabinoids can be delivered to the substrate. The liquids and powders can be delivered in the same quantity or in different quantities. Accordingly, the ratio of one or more cannabinoids relative to one or more other cannabinoids can be controlled. In other examples, the liquid or powder to be delivered to the substrate can include a plurality of cannabinoids, either in equal proportions or in desired ratios. Thus, a single liquid or powder can be delivered to the substrate to deliver either a single cannabinoid or a plurality of cannabinoids. The plurality of cannabinoids delivered to the food product with one or more powders or liquids can include greater than one cannabinoid up to the full range of cannabinoids, such as approximately 113 cannabinoids.

Because the cannabinoid is added to the edible product after the edible product has been prepared, it should be appreciated that the cannabinoid is not subjected to the food preparation process. Thus, the cannabinoid is not subjected to the mixing of ingredients of the food product, the cooking of the food product, the freeze drying of the food product, the dehydration of the food product, or the like. As a result, the active pharmaceutical ingredient is not subject to processes that might otherwise degrade the efficacy of the active pharmaceutical ingredient. The present disclosure recognizes, however, that the methods of delivering the cannabinoid to prepared food product can further be applied to raw food product, such as raw fruit and nuts, and other non-edible substrates.

According to another aspect, and API such as cannabis or other formulations may be applied via any suitable printing process, including 3D, inkjet printing, or any suitable alternative printing process. In some cases, such printing can be used to apply, for example, any suitable label such as a cannabis warning symbol or warning, and the ink dots used in the printing could be composed largely of a targeted formulation derived from cannabis or hemp. In some aspects, formulations used for precise addition to edible products may comprise a substantial fraction of a plurality of any suitable cannabinoids. Non-limiting examples of such cannabinoids include THC or CBD, or of a combination of one or both of these and other cannabinoids. In some aspects, a mix of cannabinoids can be supplemented by one or more terpenes or flavonoids, which terpenes and/or flavonoids could be extracted from cannabis or hemp, or could be provided as pure substances acquired or synthesized commercially from other sources. Further, bitter or strong cannabis flavors may be hidden within the much more prevalent flavor of the "host" edible product. Alternatively or additionally, the at least one cannabinoid can be deposited on a location of a product that is not designed to be brought into direct initial contact with the tongue during ingestion, thereby further masking the taste of the at least one cannabinoid. For instance, the at least one cannabinoid can be applied to the top rounded surface of a cookie, it being recognized that cookies are designed to be placed into the mouth with the bottom flat surface against the tongue. Alternatively or additionally, the microdroplets can be coated with a sugar or other suitable taste-masking agent as desired.

In some aspects, soft edible products such as chocolate, gummies, licorice, and the like can be used as a "carrier" or "host" for a quantity of cannabinoid that can be injected into the soft edible product (by air gun, or by needle, or by other suitable methods known in the art), to push the added material into the bulk of the soft edible product. Cannabis flavors may be masked by such an approach. In some aspects, energy such as infrared light, forced air, or microwaves may be applied to a surface of an edible product to soften (or further soften) the material in a small area, and cannabis or hemp-derived material may be injected into the area pre-treated with infrared more easily (or to a great depth in the host edible product). The energy can be applied before injection, after injection, or both before and after injection. In other examples, the at least one cannabinoid can be applied to multiple surfaces of the edible product up to all surfaces of the edible product.

For edible products, for instance candies, hard or soft, or any other suitable edible substrate, a visible designs with CBD, THC, or other cannabinoid formulations acting as the ink can be printed on the item. One may print microdots having an indication of the dosage of the cannabinoid formulation as desired. Doses may also be sprayed on, or added as an additional layer, such as a candy or chocolate layer. In some aspects, the at least one cannabinoid can be mixed in with food ingredients, particularly when such ingredients are suitable for addition, shortly prior to packaging or delivering a prepared edible product (for example, as an ingredient in an icing or other coating, or as part of the sugar coating applied to gummy candies.

In some aspects, an edible product can be coated with small oil spheres or a coating of solid powder, each containing the at least one cannabinoid, to block taste or hide cannabis flavor. In some aspects, colorant may be added to the at least one cannabinoid prior to delivering the at least one cannabinoid to the edible product, in order to blend the formulation blend with the coloring of the edible product.

According to an aspect, the applicator sprays the API onto a surface of the edible product; wherein the cannabis or hemp-derived material either diffuses into or remains on the surface of the cannabis or hemp-derived material.

According to an aspect, the applicator shoots the API-containing liquid into the edible product.

According to an aspect, the applicator squirts the API-containing liquid into the edible product.

According to an aspect, the applicator squirts the API-containing liquid onto a surface of the edible product.

According to an aspect, the applicator stamps API-containing material onto a surface of the edible product.

According to an aspect, the applicator prints API-containing material onto a surface of the edible product.

According to an aspect, the applicator sprinkles API-containing material onto a surface of the edible product.

According to an aspect, the applicator builds one or more API-containing micro-pills on a surface of the edible product.

According to an aspect, the applicator adds a conformal coating of the API-containing material to the edible product.

According to an aspect, the applicator encapsulates or mixes the API-containing material with one or more modifiers that are configured to modify at least one or more of flavor, mechanical properties, or aesthetics of applied cannabis or hemp material before adding to the edible product.

According to an aspect, the applicator adjusts size, location, or distribution of the API-containing material on the edible product to modify flavor or aesthetics.

According to an aspect, the applicator adjusts one or both of a concentration of the API in a solution or an ingredient of the solution.

According to an aspect, energy is added to a surface of the edible product to increase adhesion of the API-containing material to the edible product. According to an aspect, energy is added to increase a temperature at the surface. In one aspect, the temperature can be increased by directing at least one of forced air, microwaves, and light, such as infrared light, to the surface. The energy can be added prior to delivering the API-containing material to the edible product, after delivering the API-containing material to the edible product, or both before and after delivering the API-containing material to the edible product.

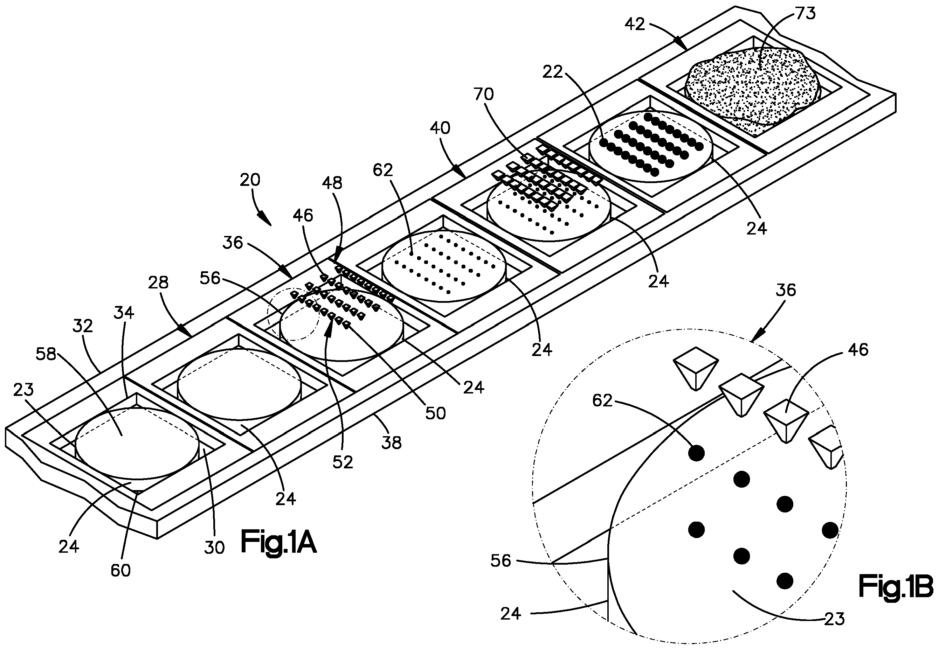

Referring now to Figs. 1A-2, all of the above method steps and apparatus described herein, including an active-containing substrate, can be incorporated into or provided by any suitable system. While one such system 20 is illustrated and described herein, it is recognized that numerous alternatives are available for dispensing an approximate dose of the active pharmaceutical ingredient onto or into a desired substrate as described above. In one example, the system 20 is configured to deliver an active pharmaceutical ingredient 22 to a substrate 23, which can be configured as an edible product 24, thereby producing an active-containing substrate. When the substrate is an edible product, the active-containing substrate can be referred to as an active-containing edible product. As described above, the edible product 24 can be any suitable fully prepared food product. Further, as described above, the active pharmaceutical ingredient can include at least one cannabinoid, at least one alternative drug or material that provides a health benefit or recreational drug experience, or any desired alternative ingestible controlled substance as designated by law. It will be appreciated that the system 20 can provide a cost-effective and efficient method for providing a product line of substrates having desired amounts and types of active pharmaceutical ingredients.

In some instances, it may be desirable to add one or more auxiliary edible products to the prepared edible product 24, either before or after delivering the active pharmaceutical ingredient 22 to the edible product 24. Examples include adding icing to a cookie, or frosting to a brownie or cake. However, in these examples, the cookie and brownie can have been fully cooked or otherwise prepared prior to adding the pharmaceutical ingredient. The system 20 can include one or more up to all of a delivery station 28 that is configured to receive one or more edible products, a dosing station 36 configured to deliver an approximate dose of the active pharmaceutical ingredient (API) 22 to the one or more edible products, a post-processing station 40, and a packaging station 42 that can be configured to package the edible product 24 that carries the approximate dose of the active pharmaceutical ingredient 22. In some examples the approximate dose can be a precise dose as described herein. The post-processing station 40 can be configured to at least one of 1) dries a solvent, for instance, when the API is delivered as a solution, 2) increases a viscosity of the API, 3) further adheres the API to the substrate, 4) disperses the API along the substrate, and 5) increases absorption or diffusion of the API into the substrate. Operation of the system can be controlled by any suitable controller, such as the Champion 3700 Digital Dispensing Benchtop System commercially available from Creative Automation Company, having a place of business in Sun Valley, CA.

The terms "substantial," "approximate, "about," and words of similar import when used with respect to a quantity, volume, mass, weight, dosage, size, shape, direction, or other parameter, include the stated parameter specifically along with ranges within plus and minus 20% of the stated parameter, for instance plus and minus 10% of the stated parameter, including within plus and minus 5% of the stated parameter, such as within plus and minus 2% of the stated parameter, including plus and minus 1% of the stated parameter.

When the at least one active pharmaceutical ingredient is to be delivered as an API-containing liquid 25, which can be a solution of the type described above or a pure API for instance as an oil, the system 20 can include a holding tank 26 that is configured to retain the liquid 25. Thus, while the liquid 25 can be a pure cannabis extract in one example, in other examples the cannabis extract can be mixed or otherwise combined with one or more other materials as desired, such as a solvent. In one example, the liquid is a solution having an approximate concentration of the cannabinoid or other active pharmaceutical ingredient 22 described above. The approximate concentration of the active pharmaceutical ingredient in the API containing material can be a known concentration described above. Thus, the active pharmaceutical ingredient 22 can define the solute of the solution, and the solution can define any suitable solvent. In one example, the solvent can be an alcohol, such as ethanol or any alternative alcohol as desired, or any other viscosity reducing agent as desired. In one example, the liquid 25 can contain a concentration of the active pharmaceutical ingredient that is in a range from approximately 40% to approximately 90%, such as from approximately 50% to approximately 70% by volume in solution with the solvent. It is recognized that the solvent will be removed substantially in its entirety during a subsequent drying step. For instance, the solvent can easily evaporate after being applied to the edible product 24. Nevertheless, it may be desired for the solvent to be safe for consumption in trace amounts.

Alternatively, the cannabinoid or other active API-containing liquid 25 can be a stand-alone extract, meaning that it is not mixed with a carrier that is designed to be burned or otherwise evaporated off. The extract can be purified, partially purified, or unpurified as desired. It is recognized that such stand-alone extracts can be in the form of a resin having a relatively high viscosity that can prevent the extract from being suitably free flowing for easy delivery to the substrate 23. Therefore, as described in more detail below, the system 20 can include one or more heaters that are configured to raise the temperature of the extract, thereby lowering the viscosity of the API-containing liquid. Alternatively or additionally, an additive such as alcohol can be added to the liquid 25 that lowers the viscosity of the liquid. The alcohol readily evaporates after the liquid 25 has been applied to the substrate 23. It is recognized that the extract having a suitably low viscosity can be readily delivered to the substrate in any manner described herein. For instance, it can be desirable to maintain the extract at a heated temperature during application of the extract to the substrate 23. The heated temperature can range from about 100 degrees F to about 200 degrees F, such as about 150 degrees F to approximately 180 degrees F. Although it is envisioned that the extract has a sufficiently low viscosity at room temperature, it may nevertheless be desirable in some instances to maintain the solution at the heated temperature. Because the approximate dose of the at least one cannabinoid in the liquid 25 is known, a predetermined approximate volume of the liquid 25 delivered from the holding tank 26 to the dosing station 36, and thus to the edible idem 24, can contain approximately a predetermined approximate desired dose of the pharmaceutical ingredient 22.

The delivery station 28 can be configured to receive a plurality of substrates 23 such as a plurality of edible products 24. This, while the substrates are illustrated as edible products 24, it is recognized that the substrates can be configured as any suitable alternative substrate as described above. In one example, the system 20 includes one or more support surfaces 30 of at least one support member 32 that are configured to receive and support a respective one or more edible products 24. The support surfaces 30 can be defined by respective predetermined locations of the support member 32. The predetermined locations can be defined by geometric markings. Alternatively or additionally, the predetermined locations can be defined by pockets 34 that are defined by the support member 32. At least one or both of the support member 32 and the dosing station 36 can be movable so as to bring the dosing station 36 into alignment with the edible product 24. The dosing station 36 can be configured to deliver the approximate volume of the liquid 25 to one edible food product at a time, or can be configured to deliver a plurality of approximate volumes of the liquid 25 to a respective plurality of edible food products simultaneously. In this regard, descriptions herein of singular elements apply with equal force and effect to a plurality of the singular element and at least one of the singular element. Thus, the term "a," "an," "the" as used herein in connection with a singular apparatus or method step includes a plurality of the apparatus or method steps and at least one of the apparatus or method steps. Conversely, descriptions herein of plural elements apply with equal force to the singular element, or at least one of the singular element. Thus, a plural apparatus or method steps described herein includes the singular "a," "an," and "the," as well as "at least one." The dosing station 36 can be configured as an ultra-low volume liquid handling machine commercially available from Biofluidix having a place of business in Freiburg, Germany.

In one example, the support member 32 can be configured as any suitable delivery member such as a conveyor 38 or other suitable support member that is designed to support and transport the edible products to be brought into operative alignment with the dosing station 36. The conveyor 38 can be movable so as to correspondingly transport the edible product 24 from the delivery station 28 to the dosing station 36. Alternatively, the support surface 30 can be stationary, and the dosing station 36, including the applicator which can be configured as one or more dosing heads as described below, can be movable to be brought into alignment with the substrate 23. Alternatively still, both the support surface 30 and the dosing station can be movable so as to bring the dosing heads into alignment with the substrate 23. Thus, it can be said that at least one of the support surface 30 and the dosing station 36 can be movable with respect to the other of the support surface 30 and the dosing station 36 so as to bring the substrate 23 into alignment with the dosing heads of the dosing station 36.

Alternatively still, it is recognized that the system 20 can be configured for self-service whereby a user places the substrate onto the support surface 30 at the dosing station 36. Alternatively, the user can place the substrate 23 onto the support surface 30, and manually moves the substrate 23, for instance along the support surface 30, to the dosing station 36. In this example, the support surface 30 can be a stationary support surface. Further, the dosing station can be stationary.

After the active pharmaceutical ingredient has been delivered from the dosing station 36 to the substrate 23, the active-containing substrate 23 can be moved from the dosing station 36 to the post-processing station 40. The active-containing substrate 23 can be moved from the dosing station 36 to the post-processing station 40 using the support surface 30 or any suitable alternative apparatus. In this regard, the post-processing station 40 can be positioned inline with the dosing station 36 along the support surface 30 in some examples. Alternatively, the post-processing station 40 can be offline with respect to the support surface 30. Thus, the active-containing substrate can remain at the post-processing station 40 for as much time as desired until the active-containing substrate is suitable to be packaged. At that point, the active-containing substrate can be moved from the post-processing station 40 to the packaging station 42. The support surface 30 or any suitable alternative apparatus can move the active-containing substrate from the post-processing station to the packaging station 42. In this regard, the post-processing station 40 can be positioned inline with the dosing station 36 along the support surface 30, or can be offline with respect to the support surface 30.

Once the edible product 24 is aligned with the dosing station 36, the dosing station 36 is configured to deliver a predetermined approximate volume of the active pharmaceutical ingredient, such as at least one cannabinoid, to the edible product. In some examples, the active pharmaceutical ingredient can be presented as the liquid 25. Because the concentration of the active pharmaceutical ingredient in the liquid 25 is known, and the desired dose of the active pharmaceutical ingredient to be delivered to the substrate 23 is known, the approximate volume of the liquid 25 to be delivered to the substrate 23 can be determined. In some examples, electrostatic forces can be created that drive the active pharmaceutical ingredient to the substrate 23, whereby the active pharmaceutical ingredient and the substrate are oppositely charged. For instance, the substrate 23 can be provided with a negative electrical charge, and a positive charge can be applied to the liquid or powder to be delivered, thereby creating the electrostatic charge.

In other examples, it is recognized that the active pharmaceutical ingredient can be delivered to the substrate 23 as a powder. For instance, the liquid 25 containing the at least one cannabinoid can be in the initial form of a resin that can dry and crystallize. The resulting crystals can be ground into a powder having a desired dose of active pharmaceutical ingredient. Because the density of the active pharmaceutical ingredient in the powder is known, and the desired dose of the active pharmaceutical ingredient to be delivered to the substrate 23 is known, the approximate mass of the powder to be delivered to the substrate 23 by the dosing station 36 can be determined.

The dosing station 36 can include at least one applicator of the type described above, such as a plurality of applicators. Each applicator can define a dosing head 46 that is configured to dispense a respective approximate quantity of the approximate volume of the liquid 25 that is delivered from the holding tank 26. Thus, the dosing station 36 can include at least one dosing head 46 such as a plurality of dosing heads 46. The dosing station 36, and in particular the applicators and thus the dosing heads 46, is in fluid communication with the holding tank 26. Thus, the dosing heads 46 are configured to receive respective quantities of the volume of the liquid 25 delivered from the holding tank 26, and dispense the respective quantities to the edible product 24. The respective quantities dispensed by the dosing heads 46 cumulatively define the approximate volume of liquid 25 that has been received from the holding tank 26.

As will now be described, the dosing heads 46 can be configured to deliver precise quantities of the volume of the liquid 25 to the edible products 24. In some examples, the precise quantities can be microquantities applied to the edible product 24. Thus, the edible products 24 can receive a predictable dosage of the active pharmaceutical ingredient within federal regulations. Further, the dosage of the active pharmaceutical ingredient can be applied at specific locations of the edible product as desired. For instance, in certain examples, it may be desirable to deliver the active pharmaceutical ingredient such that it is substantially evenly distributed on or in the edible product 24. As a result, for instance when the edible product is a large baked good, consumption of different regions of the edible product in equal volumes will cause ingestion of substantially identical quantities of the active pharmaceutical ingredient. In one example, the dosing heads 46 can be defined by a True Volume^{™} Piston Positive Displacement Pump commercially available from Creative Automation Company having a place of business in Sun Valley, CA. In another example, the dosing heads 46 can be defined by a Pipetman M P10M device commercially available from Gilson Inc., having a place of business in Middleton, WI.

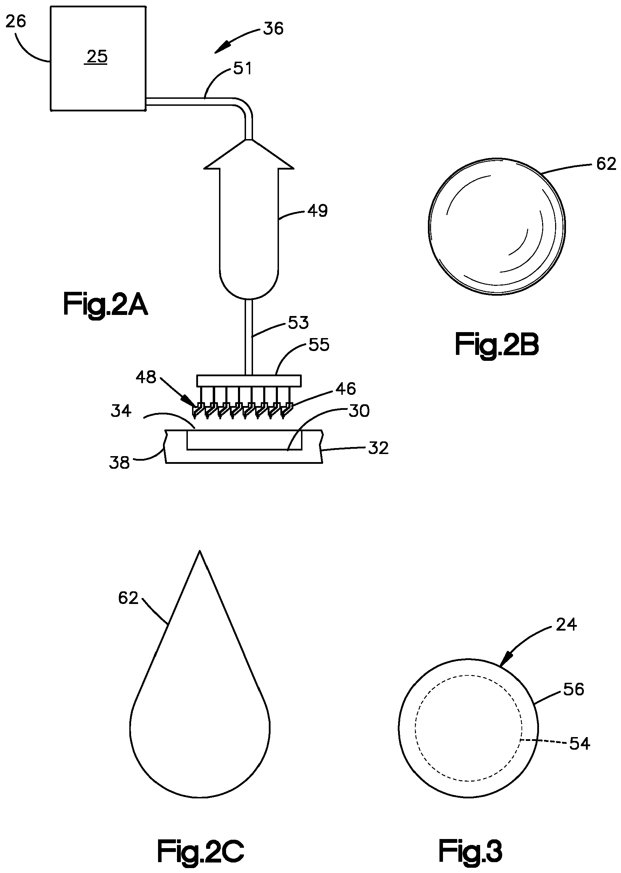

Referring now to Fig. 2 in particular, the dosing station 36 can include an injection reservoir 49 disposed between the holding tank 26 and the dosing heads 46. The dosing station 36 can include a first conduit 51 that extends from the holding tank 26 to the reservoir 49, and a second conduit 53 that extends from the reservoir 49 toward the dosing heads 46. Thus, the reservoir 49 can receive the volume of the liquid 25 from the holding tank 26. The dosing station 36 can further include a second conduit 53 that extends from the reservoir 49. The second conduit 53 can extend to a manifold 55. The reservoir 49 can thus deliver the volume of the liquid 25 to the manifold 55 under a pressure differential provided by a pump, and the manifold 55 can distribute the volume of liquid 25 to the dosing heads 46. In this regard, it should be appreciated that the second conduit 53 is in fluid communication with the dosing heads 46. The pump can be a positive pump that defines a positive pressure differential. The holding tank 25 can put under positive pressure so as to provide a positive force that urges the liquid 25 out of the holding tank 26 toward the dosing heads 46. Alternatively, the holding tank 25 can put under negative pressure so as to draw the liquid 25 out of the holding tank 26 toward the dosing heads 46. In other examples, the system 20 can include a plurality of pumps that are each configured to provide a respective pressure differential to a respective one or more of the dosing heads 46.

The pumps can, for instance, define respective pistons that are movable in corresponding cylinders to eject a predetermined precise volumes of the liquid 25. In this regard, the stroke length of the piston that delivers the liquid 25 to a first at least one dosing head 46 can be different than the stroke length of the piston that delivers liquid 25 to a second at least one dosing head 46. Alternatively, the pumps can include an elastic micropipe with an inner diameter that is partially squeezed by a piezo stack actuator so as to drive the liquid 25 out of the dosing head 46.

In some examples, different dosing heads 46 can be configured to deliver different quantities of the respective volume of liquid 25 to the edible product 24 (see Fig. 1). Further, the liquid 25 delivered by the first at least one dosing head 46 can include a different active pharmaceutical agent than the liquid 25 delivered by the second at least one dosing head 46. Further still, the system 20 can be configured to deliver any number of API-containing liquids 25 each containing a different pharmaceutical agent to a respective at least one dosing head 46. Accordingly, the dosing heads 46 can combine to deliver active pharmaceutical agents from different liquid extracts in different quantities onto a common substrate 23. Alternatively or additionally, the different liquids can have different concentrations of their respective active pharmaceutical agent. The system 20 can therefore include any number of holding tanks 26 as desired, each tank containing a different liquid extract that contains a different at least one pharmaceutical active ingredient. The different liquid extracts can be delivered to different respective ones of the dosing heads 46. Thus, different dosing heads can be configured to deliver different cannabinoids to the substrate.

As one example, a first group of dosing heads 46 can be configured to deliver a dose of a first active pharmaceutical agent, and a second group of dosing heads 46 can be configured to deliver a dose of a second active pharmaceutical agent, wherein the second active pharmaceutical agent is different than the first active pharmaceutical agent. For instance, the first active pharmaceutical agent can be THC, and the second active pharmaceutical agent can be CBD. Further, the first active pharmaceutical agent can be delivered in a different predetermined approximate dose than the second active pharmaceutical agent. Further still, the tank containing the first active pharmaceutical agent can be maintained at a different temperature than the second tank. Thus, the viscosity of each of the respective API-containing liquids 25 can be individually controlled. Additionally, the temperature of one or more up to all of the respective conduits 51 and 53 and at the respective dosing heads 46 can be different so as to individually control the viscosity of each liquid extract as it travels from the respective tank to the respective one or more dosing heads 46.

The system 20 can include any suitable feedback mechanism to provide an indication that the at least one dosing head 46 has delivered the at least one active pharmaceutical ingredient to the substrate 23. The feedback mechanism can be a closed feedback loop in some examples. For instance, a pressure sensor can be placed in the conduit 53 so as to measure the backpressure in the conduit 53. A drop in the backpressure, for instance, can indicate that the respective at least one dosing head 46 has delivered the respective at least one active pharmaceutical ingredient to the substrate 23. Alternatively, the system 20 can include a load cell that determines, by sensing weight, that the substrate 23 is in alignment with the dosing head 46. Alternatively still, the system 20 can include a visual recognition system that includes a visual sensor to visually identify that the substrate 23 is in alignment with the dosing head 46. It is therefore appreciated in some examples, that the substrates 23 can be positioned at any location on the support surface that need not be a predetermined location of the support surface.

Further, the system 20 can include a camera that is designed to measure a quantification of the microdroplets delivered from the dosing heads 46. For instance, the camera can measure a cross-sectional dimension of the microdroplets as they travel from the dosing heads 46 to the substrate 23. It is recognized microdroplets can be elongated as they travel out of the dosing heads. However, the surface tension of the microdroplets can cause the microdroplets to become more spherical as they travel from the dosing heads 46 to the substrate. Thus, in one example, the cross-sectional dimension can be a maximum cross-sectional dimension that approximates the diameter of a sphere, such that an approximation of the volume of the microdroplets can be calculated if desired. However, the cross-sectional dimension can be any suitable alternative cross sectional dimension that has a relationship to the volume of the microdroplet. The cross-sectional dimensions or calculated approximations of the volumes of the microdroplets can be compared to each other so as to ensure consistency of the volume of microdroplets being delivered to the substrates 23, or to verify a desired variation in the volumes of microdroplets. The cross-sectional dimensions or calculated approximations can then be integrated into the feedback look to ensure proper operation of the system 20. In one example, the camera can be a SmartDrop System commercially available from Biofluidix having a place of business in Freiburg, Germany.

As described above, the system 20 can be configured to deliver heat to the liquid 25 either in one or more of the conduits and/or in the dosing head 46 prior to or during dispensing of the API containing liquid to the substrate 23. The heat can be sufficient to decrease the viscosity of the API-containing liquid 25. In some examples, for instance when the API-containing liquid 25 includes a solvent, the step of delivering heat to the liquid 25 can cause the solvent to evaporate, such that pure API having a sufficiently low viscosity is dispensed from the dosing heads 46. Thus, in one example, the API-containing liquid 25 can include the API and solvent, and can travel from the holding tank 26 to the dosing head 46. The API-containing liquid 25 can be heated between the holding tank 26 and the dosing head 46 to decrease the viscosity of the liquid 25 and, in some instances, evaporate some or all of the solvent. Alternatively or additionally, the API-containing liquid 25 can be heated at the dosing head 46 so as to decrease the viscosity of the liquid 25 and, in some instances, evaporate some or all of the solvent. In one example, the system 20 can include at least one heater that delivers heat to one or more up to all of the first conduit 51, the second conduit 53, the injection reservoir 49, the manifold 55, and the dosing head 46, so as to decrease the viscosity of the API-containing liquid and, in some instances, evaporate the solvent. In one example, the liquid 25 can be maintained at a temperature in a range from approximately 100 degrees F to approximately 200 degrees F, such as from approximately 140 degrees F to approximately 200 degrees F, and in one example from approximately 150 degrees F to approximately 180 degrees F. Alternatively, in some examples such as when the liquid 25 is a solution, the liquid 25 can be maintained at room temperature.

While the dosing heads 46 can be configured to deliver to the substrate 23 the liquid 25 that contains at least one active pharmaceutical ingredient in one example, the dosing heads 46 can alternatively be configured to deliver the at least one active pharmaceutical ingredient to the substrate 23 in the form of a solid or powder in the manner described herein with respect to the liquid 25. Thus, examples above of applying the active pharmaceutical ingredient in the form of a liquid can apply with equal force and effect to a powder including the at least one active pharmaceutical ingredient, unless otherwise indicated. Each dosing head 46 can be configured to deliver microdroplets of the API as described above. Thus, it should be appreciated the powder can be delivered to the substrate 23 as a microquantity. The powder can be stored in the holding tank 26, and can be directed through the first conduit 51 and the second conduit 53 to the dosing head 46, either directly or through the manifold 55. Thus, it can be said that a quantity of API containing material can be applied to the substrate 23. The API containing material can be in the form of a powder or a liquid. Thus, the API containing material can include a desired concentration of active pharmaceutical ingredient as described above. In other examples, the API containing material can include only the active pharmaceutical ingredient.

Further still, while each of the dosing head 46 can be configured to dispense the API-containing liquid 25 that has been received from the holding tank 26, it is recognized that the API-containing liquid 25 can be delivered using other methods. For instance, the system 20 can include a first holding tank that contains the API in liquid or solid form, and a second holding tank that contains a solvent. The API and solvent can mix at the dosing station 36. For instance, the API and solvent can mix at the dosing head 46. In one example, the dosing head can include a first chamber that receives the API, and a second chamber that receives the solvent. The API and solvent can mix in the dosing head 46 to produce a solution having the predetermined concentration of API. The solution produced in the dosing head 46 can then be dispensed as one or more microdroplets in the manner described herein. In some examples, the concentration can be varied inside the dosing head 46. That is, the respective proportions of API and solvent that are mixed in the dosing head 46 can be varied. Further, the API or the solution can be mixed with at least one other ingestible modifier that is configured to modify at least one of flavor, one or more mechanical properties, or one or more aesthetics of the cannabis or hemp material. The mixing can occur in the dosing head 46 or at any other location as desired. For instance, the at least one other edible product can be mixed in the liquid 25 in the holding tank 26 in some examples.

Referring again to Figs. 1A-2B, in one example, the dosing heads 46 can be arranged in an array 48 that includes at least one row 50 of dosing heads 46. The dosing heads 46 of each row 50 can be substantially equidistantly spaced along the respective row 50. Alternatively, the dosing heads 46 can be variably spaced along the respective row 50. The array 48 can further include a plurality of columns 52 that spaces the rows 50 from each other. The dosing heads 46 can be equidistantly spaced along the respective columns 52. Alternatively, the dosing heads 46 can be variably spaced along the respective columns 52. In one example, all of the dosing heads 46 can be configured to deliver the same at least one active pharmaceutical ingredient. Alternatively, as described above, different groups of the dosing heads 46 can be configured to deliver a respective different active pharmaceutical ingredients. Each group can include at least one dosing head 46 up to a plurality of the dosing heads 46. Each group can be defined by a respective one or more of the rows 50. Alternatively, each group can be defined by a respective one of the columns.

Referring now to Figs. 1A-3, the dosing heads 46 can be aligned with different respective locations of a dosing zone 54 the edible product 24. Accordingly, the dosing heads 46 can be positioned to deliver their respective quantities of the volume of liquid 25 to the different respective locations of the dosing zone 54. Further, the system 20 can be configured to deactivate select dosing heads 46 that are out of alignment with the dosing zone 54 and thus do not receive respective portions of the volume of liquid 25, and activate select dosing heads 46 that are aligned with the dosing zone 54 and thus receive respective portions of the volume of liquid 25. In some examples, the system 20 can include a sensor that identifies the dosing zone 54 of the edible product 24. The sensor can be a camera, a weight sensor that measures the weight of the substrate 23 on the support surface and determines the dosing zone based on the weight and/or size, or any suitable alternative sensor. The dosing zone 54 can be at least partially defined by an outer perimeter 56 of the edible product 24. For instance, the dosing zone 54 can be defined in its entirety by the outer perimeter 56 of the edible product 24. Thus, an entire outer surface of the edible product 24 can define the dosing zone 54. In some examples, the dosing zone 54 can be disposed inside the outer perimeter 56 in its entirety. For instance, the dosing zone 54 can be greater than half, for instance greater than 75%, of a footprint defined by the outer perimeter. Either way, it can be said that the dosing zone 54 can be a substantially predetermined location with respect to the outer perimeter 56 of the edible product 24. Thus, the dosing zone 54 can be consistent among a plurality of differently sized edible products 24, such as cookies or brownies that can have similar but non-identical sizes and shapes.

The dosing heads 46 can be spaced from each other as desired so as to deliver a desired distribution of the active pharmaceutical ingredient to the edible product 24 in the dosing zone 54. Alternatively, one or more dosing heads 46 can be movable so as to deliver the active pharmaceutical ingredient to multiple locations of the edible product 24. In one example, the dosing heads 46 are configured to deliver a substantially even distribution of the volume of liquid 25 to the edible product 24 in the dosing zone 54. For instance, the respective quantity of the volume of suspension dispensed by each of the dosing heads 46 or each plurality of dosing heads can be substantially equal to the respective quantity of suspension dispensed by the other dosing heads 46 or other pluralities of dosing heads 46.

In another example, the system 20 can divide the dosing zone 54 into a plurality of subzones. Each subzone can be configured to receive a different at least one active pharmaceutical ingredient. Thus, a first group of at least one dosing head 46 can deliver a first at least one active pharmaceutical ingredient to a first one of the subzones, and a second group of at least one dosing head 46 can deliver a second at least one active pharmaceutical ingredient that is different than the first at least one active pharmaceutical ingredient to a second one of the subzones. Alternatively or additionally, the first group of at least one dosing head 46 can be configured to deliver a first dose of the first at least one active pharmaceutical ingredient, and the second group of at least one dosing head 46 can be configured to deliver a second dose of the second at least one active pharmaceutical ingredient that is different than the first dose. In still another example, the first and second groups of at least one dosing head 46 can be configured to deliver the same at least one active pharmaceutical ingredient, but in different doses. The active pharmaceutical ingredient can be substantially evenly distributed in each of the subzones.

In some examples, at least one dosing head 46 such as a plurality of dosing heads 46 can be movable along the substrate 23 so as to deliver the respective at least one active pharmaceutical ingredient at different locations of the edible product 24. Further, the dosing heads 46 can be configured to deliver different active pharmaceutical ingredients to the substrate 23. For instance, the dosing heads 46 can be configured to deliver different combinations of liquids and/or powders. In one example, the dosing heads 46 can deliver to the substrate 23 a first liquid or powder that contains a first active pharmaceutical ingredient. Next, the dosing heads 46 can deliver to the substrate 23 a second active pharmaceutical ingredient that is different than the first active pharmaceutical ingredient. Next, the dosing heads 46 can deliver to the substrate 23 a third active pharmaceutical ingredient that is different from each of the first and second active pharmaceutical ingredients, and so forth until all desired active pharmaceutical ingredients have been delivered to the substrate 23.

When the dosing heads 46 are arranged in groups of dosing heads 46 that each deliver a respective different at least one active pharmaceutical ingredient, the different active pharmaceutical ingredients can be delivered to respective different locations of the substrate 20. For instance, the dosing heads 46 can remain stationary with respect to the substrate 23 as the active pharmaceutical ingredient is delivered to the substrate 23. Alternatively, the dosing heads 46 can be movable along the substrate 23, such that the combination of active pharmaceutical ingredients as delivered by different groups of at least one dosing head 46 can be delivered to the same respective location of the substrate 20. The heads 46 can be movable such that the dosing heads 46 can deliver the respective active pharmaceutical ingredient to different respective locations of the substrate 23 than the other dosing heads. The active pharmaceutical ingredients in the different respective locations can be substantially evenly distributed in at least one direction along to the substrate 23. For instance, the active pharmaceutical ingredient in the different locations can be substantially evenly distributed in two perpendicular directions along the substrate 23.

The substrate 23 includes an external surface that defines an inner surface 60 that faces the support surface 30, and an outer surface 58 that is opposite the inner surface 60. The dosing heads can deliver the active pharmaceutical ingredient to the outer surface 58 of the substrate 23. The edible product 24 defines a thickness that extends from the inner surface 60 to the outer surface 58. The delivered volume of active pharmaceutical ingredient can substantially remain on the outer surface 58. Delivering the volume of liquid to the outer surface 58 can expose the liquid to oral receptors, thereby increasing speed of ingestion of the active pharmaceutical ingredient. Alternatively or additionally, the delivered volume of liquid 25 can permeate through the outer surface 58 so as to impregnate at least a volume of a thickness of the edible product that extends from the outer surface 58 to the opposed inner surface 60. Alternatively still, the active pharmaceutical ingredient can be injected into the substrate 23 between the inner surface 60 and the outer surface 58. For example, at least 20% of the active pharmaceutical ingredient can be disposed in a middle 75% of the thickness. The middle 75% of the thickness can be equidistantly spaced from each of the inner surface 60 and the outer surface 58. For instance, at least 20% of the active pharmaceutical ingredient can be disposed in a middle 50% of the thickness. The middle 50% of the thickness can be equidistantly spaced from each of the inner surface 60 and the outer surface 58. In some examples, the distribution along the outer surface of the substrate 23 can be different than the distribution along the thickness of the substrate 23 from the outer surface to the inner surface.

In one example, the dosing heads 46 can be configured to deliver the respective quantities of the active pharmaceutical ingredient to the respective locations of the outer surface 58 of the edible product 24 in the form of microdroplets 62. The microdroplets 62 can have any suitable size and shape as desired. In one example, the microdroplets 62 can including microquantities of the active pharmaceutical ingredient. For instance, the microdroplets 62 can define a maximum cross-sectional dimension along a horizontal direction that is in a range from approximately 5 millionths of an inch, for instance when printed, up to approximately 100 thousandths of an inch. For instance, the range can be from approximately 5 thousandths of an inch to approximately 50 thousandths of an inch. In one example, the maximum cross-sectional dimension along the select direction can be in a range from approximately 20 thousandths of an inch to approximately 40 thousandths of an inch. The dosing heads 46 are spaced from the edible products 24 along a direction of travel of the active pharmaceutical ingredient from the dosing heads 46 to the edible products 24. Thus, the active pharmaceutical ingredient is delivered to the substrate along the direction of travel. The select direction can be substantially perpendicular to the direction of travel. In one example, the dosing heads 46 are spaced above the edible products 24 along a vertical direction. Thus, the select direction can be a substantially horizontal direction. For instance, the dosing heads 46 can be spaced from the edible products 24 any suitable distance when delivering the active pharmaceutical ingredient to the edible products 24, such as from approximately 2mm to approximately 25 mm. As shown at Fig. 2B, at least some of the microdroplets 62 up to all of the microdroplets 62 can be substantially spherical shaped. Alternatively or additionally, as shown at Fig. 2C, at least some of the microdroplets 62 up to all of the microdroplets 62 can be elongated, for instance substantially teardrop shaped or alternatively shaped as desired.

In one example, the microdroplets 62 are delivered from the dosing heads 46 to the respective locations of the edible product 24 under any suitable force, such as gravitational forces, electrostatic forces, or the like. In another example, the microdroplets 62 are delivered from the dosing heads 46 to the respective locations of the edible product under positive pressure. In this regard, the dosing station 36 can control whether the microdroplets 62 remain on the outer surface 58 of the edible product 24, and whether the microdroplets 62 permeate through the outer surface 58 into the thickness of the edible product 24 in the manner described above. In still other examples, one or more of the dosing heads 46 can be coupled to a respective needle that can be driven into the edible product 24 so as to deliver the respective quantity of the volume of liquid 25 into the edible product 24 at a location between the outer surface 58 and the inner surface 60. In some instances, the needle can be heated at a temperature suitable to soften or melt locations of the substrate contacted by the needle, in order to assist in the injection of the needle into the substrate. The heated needle can also maintain a desired viscosity of the at least one active pharmaceutical ingredient as the active pharmaceutical ingredient is being delivered through the needle and into the substrate. Whether the active pharmaceutical ingredient is delivered to the edible product 24 as microdroplets or as an injection, the active pharmaceutical ingredient can be delivered to the edible product in microquantities.

As described above, the system 20 can include the post-processing station 40 that is configured to process the edible product 24 after the liquid 25 has been delivered to the edible product 24. The post-processing station 40 can be configured to dry the solvent, for instance, when the API is delivered as a solution. In this regard, the post-processing station 40 can include any suitable drying member, such as at least one drying head 70 or a plurality of drying heads 70 that are configured to deliver a drying agent to the respective locations of the edible product 24 so as to dry the liquid 25. It is appreciated that when the liquid 25 dries, the solvent of the delivered volume of liquid 25 that carries the active pharmaceutical ingredient also dries and can evaporate, leaving the active pharmaceutical ingredient on the substrate 23. In this regard, the drying heads 70 can be arranged in an array that has an equal number of rows and columns as the array of dosing heads 46. Further, the relative position of the drying heads 70 with respect to the other drying heads 70 can be the same as the relative position of the dosing heads 46 with respect to the other dosing heads 46. Thus, the drying heads 70 can be aligned with the active pharmaceutical ingredients that were delivered to the edible product 24 by the dosing heads 46.

The drying agent can be configured as any suitable light, including ultraviolet, laser, infrared, or the like. Alternatively, the drying agent can be a forced gas that is delivered to the outer surface of the edible product 24. The forced gas can be air, nitrogen, or any suitable alternative gas such as an inert gas. The forced gas can be heated, and can have a temperature that is in a range for instance from about 100 degrees F to about 250 degrees F. Alternatively, the forced gas can be substantially unheated, and thus at ambient temperature. Alternatively still, the forced gas can be cooled, and thus at a temperature below ambient temperature. In this regard, the cooled forced gas can cause a cannabinoid to freeze on the surface of the substrate, or to delay evaporation of the solvent so as to allow the cannabis-containing solution to further impregnate the thickness of the substrate 23. Alternatively, the post-processing station 40 can expose the dosed substrate to ambient air or a controlled environment so as to dry the volume of liquid 25. It is recognized that the drying agent applied to the API can increase the viscosity of the API. The post-processing station can further cause the API to further adhere to the substrate 23. For instance, increasing the viscosity can cause the API to further adhere to the substrate 23. Further, applying forced air to the substrate 23 can cause the API disperse along the substrate as the API travels along the outer surface of the substrate 23, thereby facilitating absorption of the API into the substrate 23. For instance, it is recognized that the API can become saturated in the portion of the substrate 23 that underlies the delivered microdroplets. Causing the API to move along the outer surface of the substrate 23 then allows the API to absorb into the substrate 23 at locations of the substrate 23 that are not saturated with the API. The post-processing station 40 can further cause the API to solidify on or in the substrate 23. In some examples, the API can crystallize on or in the substrate 23. Alternatively, the API can remain as an oil on or in the substrate 23. If the at least one cannabinoid is applied as a powder, the post-processing step can apply heat to the powder, thereby causing the at least one cannabinoid to liquify on the substrate 23. Subsequent cooling of the liquified powder can cause the liquid to solidify or crystallize or otherwise adhere on or in the substrate 23.

It is appreciated that energy can be applied to the substrate 23 to improve diffusion or absorption of the API into the substrate 23. For instance, when heat is applied to the surface of certain substrates 23, and in particular certain edible products, such as chocolate, baked goods, gummy candy, lollipop, and the like, the temperature of the surface of the edible product be raised to a level whereby the edible product melts, sweats, or otherwise assumes a form that is configured to encapsulate the API. The temperature can be increased, for instance, by directing at least one of heated forced air and a light to the surface.

Once the substrate 23 has been post-processed, the active-containing edible product 24 can be transferred from the post-processing station 40 to the packing station 42. At the packaging station 42, the dried edible products 24 can be individually wrapped in any suitable package 73. Alternatively or additionally, a plurality of the active-containing edible products 24 can be wrapped in a common package. The active-containing edible product 24 can include a cooked edible product, and a dose of an active pharmaceutical ingredient carried by the cooked edible product in the dosing zone of the cooked edible product. The dose of the active pharmaceutical ingredient can be substantially evenly distributed in the dosing zone. Because the edible product was fully cooked prior to adding the active pharmaceutical ingredient, the active pharmaceutical ingredient need not be cooked after the active pharmaceutical ingredient was added.

In some examples, the edible product 24 can be configured as a plurality of nuts 37 (Fig. 4A) and/or fruits 39 (Fig. 4B) and/or a mixture of dried fruits and nuts and potentially other additional food product It is appreciated that the API is not visible in Figs. 4A-4E due to the nature of the figures. The active pharmaceutical ingredient can be applied to the nuts and fruits in any suitable manner as disclosed herein. In some examples, the nuts have been cooked, such as roasted. In other examples, the nuts can be raw. In some instances, the nuts or fruit can be prepared with salt, sugar, honey, or any suitable alternative ingredient. Thus, the nuts can be candied. In some examples, the fruit can be a raw fruit. In other examples, the fruit can be dried. In still other examples, the fruit can be candied. It is understood that fruits and nuts can have relatively low surface areas and volumes. Thus, variations in the dosage of active pharmaceutical ingredient applied to fruits and nuts can have a greater impact on the ratio of active pharmaceutical ingredient per volume of edible product when compared to edible products having larger surface areas and volumes.

Therefore, it can be particularly advantageous to precisely control the dosage of active pharmaceutical ingredient added to fruits and nuts. The active pharmaceutical ingredients can be applied to the fruits and nuts as microquantities in the manner described above, which allows for the precise control of the dosage of active pharmaceutical ingredients applied to the fruits and nuts. Depending on the size of the fruit or nut, it is recognized that microdroplets having respective volumes ranging from approximately 5 nanoliters to approximately 20 microliters can be delivered to an individual fruit or nut. Thus, each fruit or nut can include a quantity or dosage of API in the range from approximately 2.5 micrograms to approximately 20 milligrams. Therefore, each microdroplet can contain a microquantity of API in a range from approximately 0.5 micrograms to approximately 1 milligram. It is recognized, of course, that the dosage of API per dried fruit or nut can vary as desired. For instance, other quantities of microdroplets can be delivered to fruits and nuts, for instance depending on the size of the fruit and nut, the size of the microdroplet, and the concentration of API in the microdroplet. The microquantity of API in a microdroplet can allow the dosage of API delivered to a substrate to be precisely controlled as described above. Further, the dosage of API per dried fruit or nut can be precisely controlled, as can a plurality of dried fruits and/or nuts that amount to a serving. For larger edible products, such as baked goods 41 (see Fig. 4C), the microdroplets can be applied in the range of approximately 5 nanoliters to approximately 20 microliters over the entire surface and greatly increase the total API delivered to the substrate to 100 milligrams or more.

It should be appreciated that several advantages can be achieved using the system 20. In one example, the substrate 23 can include multiple active pharmaceutical ingredients, which can eliminate a conventional need to consume multiple medications each having a single active pharmaceutical ingredient. Further, microquantities of the active pharmaceutical ingredients can be applied to the substrate. Thus, the dosage of the at least one active pharmaceutical ingredient carried by the substrate can be better controlled with respect to conventional application processes. Further, the at least one active pharmaceutical ingredients can be distributed substantially evenly along the dosing zone. Further still, individual dosing of the active pharmaceutical ingredients on the substrate can allow for the use of locally produced active pharmaceutical ingredients that are applied after the substrate has been prepared, thereby avoiding the need to transport the applied active pharmaceutical ingredients across jurisdictional boundaries, which can be illegal in some jurisdictions or carry tax penalties. Further, dosing the substrate after the substrate has crossed the jurisdictional boundary can reduce or eliminate degradation of the active pharmaceutical ingredient during transportation across the jurisdictional boundary, which can sometimes involve long distances of transportation. In some examples, a dye can be used with the active pharmaceutical ingredient if desired, so as to confirm that the active pharmaceutical ingredient has been delivered to the substrate.

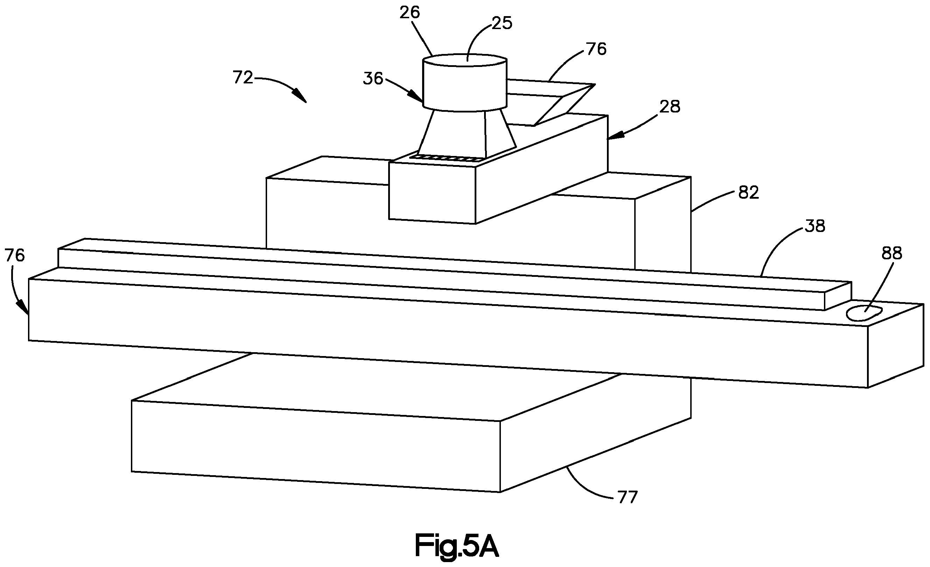

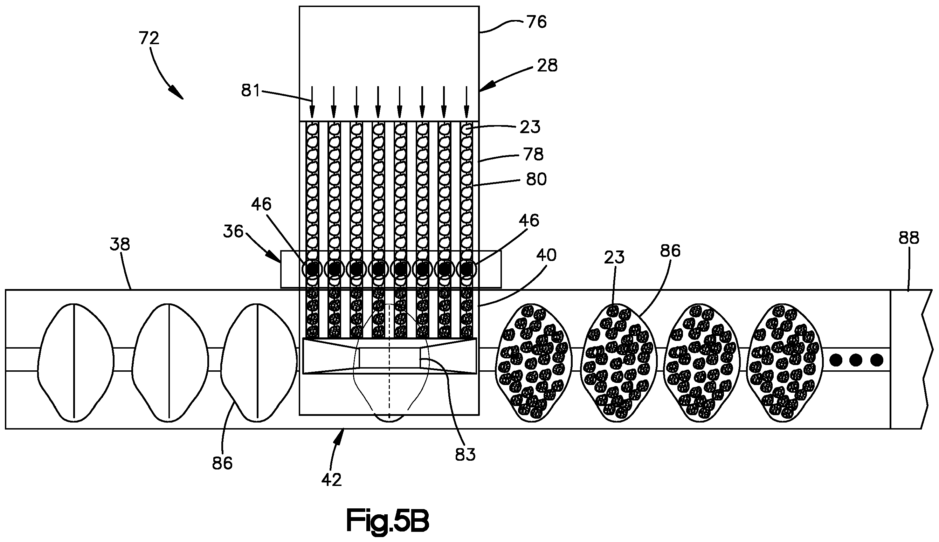

Referring now to Figs. 5A-5B, it is recognized that in some examples the system 20 described above can be configured as a single unitary stand-alone dosing machine 72. The dosing machine 72 can include the conveyor 38, the holding tank 26, the delivery station 28, the dosing station 36, the post-processing station 40, and the packaging station 42. The dosing machine 72 can further include a support structure 76 that supports the conveyor 38, the holding tank 26, the delivery station 28, the dosing station 36, the post-processing station 40, and the packaging station 42. Thus, the conveyor 38, the holding tank 26, the delivery station 28, the dosing station 36, the post-processing station 40, and the packaging station 42 can be said to be integrated into the single stand-alone dosing machine, and supported by the common support structure 76. The dosing machine 72 can further include the camera that measures the maximum cross-sectional dimension of the microdroplets in the manner described above. Further, the system 20, and dosing machine 72, can include a cleaner that is configured to remove loose particulates from the substrate 23 prior to delivering the API to the substrate 23. For instance, in the case of salted nuts, loose salt can be removed from the nuts, while salt having strong adhesion to the nuts remain. In one example, the cleaner can be configured to deliver forced air to the substrate to remove loose debris from the substrate. Thus, when the API is delivered to the substrate, the API can have strong adhesion to the substrates 23. In some examples, the forced air can be heated so as to increase the temperature of the substrate so as to improve absorption or diffusion of the API into the substrate in the manner described herein.

The delivery station 28 can include a hopper 76 or other containment member that contains one or more of the substrates 23. The delivery station 28 can further include a delivery member 78 that is configured to receive the substrates from the hopper 76, and deliver the substrates 23 from the hopper 76 to the dosing station 36. For instance, the delivery member 78 can transport the substrates 23 from the hopper 76 to the dosing station 36, and further to a third location in alignment with a delivery location whereby the dosed substrates are delivered to the conveyor 38. The delivery member 78 can include define any suitable material that can have at least one elongated groove 80 or other suitable structure that directs the substrates 23 along a respective path 81 from the hopper 76 to the dosing station 36. For instance, the delivery member 78 can include a plurality of grooves 80 that define a plurality of paths 81 from the hopper 76 to a plurality of respective dosing stations 36. Alternatively, a plurality of delivery members 78 can define respective grooves 80 that extend along respective paths from the hopper 76 to the respective dosing stations 36.

In one example, the delivery member 78 can be downwardly sloped along a direction from a first location in alignment with the hopper 76 to a second location in alignment with the dosing station 36, and further to a third location in alignment with a delivery location whereby the dosed substrates are delivered to the conveyor 38. Further, the delivery member 78 can be configured to vibrate, shake, or otherwise cause the substrates 23 to travel along the delivery member 78 from the first location to the second location, and from the second location to the third location. Alternatively, the delivery member 78 can be configured as a conveyor so as to move the substrates 23 from the first location to the second location, and from the second location to the third location. Alternatively still, as noted above, a user can manually move the substrates 23 along the delivery member 78 or otherwise move the substrates to a position in alignment with the dosing station 36.

During operation, the substrates 23 loaded into the hopper 76. The substrates 23 are then delivered from the hopper 76 to the first location of the delivery member 78. The delivery can occur under gravitational forces or any suitable alternative structure and method. In particular, the substrates 23 can be delivered to the delivery member 78 such that they are arranged along their respective paths 81. The substrates 23 can be individualized and arranged in single file on their respective delivery members 78, and thus along their respective paths 81. Alternatively, groups of the substrates 23 can be arranged on one or more of the delivery members 78. The substrates 23 traveling along the respective paths 81 can define the same type of substrate, such as fruits or nuts, or a baked good or the like. Alternatively, the different types of substrate 23 can travel along the respective paths 81. For instance, the substrates 23 traveling along one path can include dried fruits. The substrates 23 traveling along another path or the same path can include nuts, either raw or roasted. The substrates 23 traveling along still another path can include a baked good.

The substrates 23 travel along the delivery member 78 to the second location, whereby the substrates 23 are aligned with the respective a least one dosing station 36. The dosing machine 72 can include a plurality of dosing stations 36, whereby each of the dosing stations 36 is aligned with a respective one of the delivery members 78. As described above, each of the delivery members 78 causes extends along a respective path 81. Thus, each of the dosing stations 36 is aligned with one of the respective paths 81, and is configured to deliver API to the substrates 23 that travel along the respective paths 81. In one example, the substrates 23 can be arranged on the delivery member 78 such that one or more dosing heads 46 of the dosing stations 36 aligned with the respective paths are configured to deliver API to only a single substrate 23 at a time as the substrates 23 travel along the respective paths 81. In particular, the dosing heads 46 can be configured to deliver microdroplets to each individual substrate 23 in the manner described herein. Because the API is delivered in microdroplets, a precise predetermined dosage of API is delivered to each of the substrates 23.

In one example, the same API-containing liquid can delivered to a plurality of the substrates 23. Alternatively, API-containing liquids having different API characteristics can be delivered to different substrates 23. The different substrates 23 can define respective pluralities of substrates. The pluralities of substrates can travel along different respective paths 81 to different dosing stations 36 that are operably aligned with the respective paths 81. The dosing stations 36 can deliver respective APIs to the aligned substrates 23, either individually or as a group of substrates 23, where the respective APIs have at least one API characteristic that is different than the other. Alternatively, the pluralities of substrates 23 can travel along the same path 81 to the same dosing station 36. Alternatively still, the pluralities of substrates 23 can travel along different paths 81 to the same dosing station 36. The same dosing station can deliver a first API-containing liquid to a first at least one substrate 23, such as a first plurality of substrates 23. The same dosing station can deliver a second API-containing liquid to a second at least one substrate 23, such as a second plurality of substrates 23. The first and second APIs can have at least one API characteristic that is different from each other. The different API characteristic can include at least one of 1) a concentration of the API, 2) a volume of API delivered to the substrates during the delivering step, which can include at least one of a different number of microdroplets and microdroplets having different volumes, 3) a composition of the API, 4) a modifier mixed with the API, the modifier configured to modify at least one of a flavor, a mechanical property, and an aesthetics of the delivered API, and 5) a location of at least one dosing zone of the substrate 23 that defines a location of the substrate 23 where the API-containing liquid is to be deposited. The mechanical property can include viscosity of the API-containing liquid in some examples. The mechanical property can further include the surface tension of the API-containing liquid that is delivered from the dosing station. It is further appreciated that the different API characteristic can include a different predetermined dosage that is delivered to the different substrates 23. In one example, the dosage can be predetermined to correspond to a dosing regimen over a period of time. Thus, groups of one or more substrates can have dosages that differ and are designed to be consumed at predetermined times over the course of the dosing regimen. For instance, the dosage can decrease over the period of time defined by the dosing regimen. Alternatively, each substrate 23 can receive API-containing liquid 25 having the same API characteristics. Further, different groups of substrates can receive API-containing liquid 25 with at least one different API characteristic, wherein all substrates among each group receive the same API-containing liquid 25.

Alternatively, it is envisioned that a predetermined quantity of substrates 23, or a plurality of substrates 23, such as dried fruits and/or nuts, can be grouped together on the delivery member 78 along the respective path 81. Thus, the dosing stations 36 can be aligned with the plurality of substrates 23 of the group. The dosing heads 46 can thus deliver a predetermined or target quantity of API-containing microdroplets to the group as a whole as opposed to each individua dried fruit or nut. Because the API is delivered in microdroplets, a precise predetermined dosage of API is delivered to the group of substrates 23. The group of substrates 23 can be intended to be ingested in a single serving. Thus, the precise predetermined dosage is ingested when the substrates 23 of the group are ingested. It is recognized that the predetermined quantity of substrates 23 dosed in a group are not limited to fruits and nuts, but to any type of edible product 24 that is designed to be consumed in quantities, such as chips, popcorn, pretzels, candies such as gummy candy 45 (Fig. 4D), and the like. It should therefore be appreciated that the dosing station can be configured to deliver an API to at least one substrate 23 at a time, which can include the single substrate 23 or the group of substrates 23.

Each dosing station 36 can include at least one dosing head 46, such as an array of dosing heads 46, and one or more holding tanks 26 that can contain a respective API-containing liquid 25 described above. It is recognized that a plurality of dosing stations 36 can receive the API-containing liquid 25 from a common one of the holding tanks. Alternatively, the dosing stations 36 can receive API-containing liquid 25 from a different holding tank 26. The API-containing liquids 25 in the different holding tanks 26 can have different APIs from each other or the same API. Thus, the API can be a cannabinoid or any suitable alternative active pharmaceutical ingredient. Each at least one dosing head 46 of the dosing station 36 can be operatively aligned with a respective one of the paths 81 so as to deliver the active pharmaceutical ingredient to the at least one substrate 23 traveling along the respective one of the paths 81. Accordingly, as the at least one substrate 23 travels to the second location along the respective path, the dosing station 36 delivers a predetermined quantity of the API-containing liquid 25 from the at least one dosing head 46 to the respective aligned at least one substrate 23. The dosing head 46 discontinues delivery of the API-containing liquid 25 when the at least one substrate 23 has received the predetermined quantity of the liquid 25. The dosing heads 46 of the array of dosing heads 46 can combine to deliver the predetermined quantity of the liquid 25 to respective different locations of the at least one substrate 23. That is, the locations of the at least one substrate 23 can be aligned with different dosing heads 46 of the array of dosing heads 46 that are aligned with the respective path 81.

After each at least one substrate 23 has received the predetermined quantity of liquid 25, the substrates 23 move along the delivery member 78 past the second location. The dosing stations 36 resume delivery of the liquid 25 when another at least one substrate 23 has traveled to the second location to a location in alignment with the at least one dosing head 46. In this regard, the at least one substrate 23 arranged sequentially along each respective paths 81 receive the predetermined quantity of liquid 25 from the aligned one of the dosing stations 36. The predetermined quantity can be equal to each other or different than each other as desired, depending on the at least one substrate 23 and the desired dosage of the active pharmaceutical ingredient that is to be delivered to the at least one substrate 23. The dosing machine 72 can include a processor programmed with the dosage quantities that are to be applied to the substrates 23 traveling along the at least one delivery member 78. The processor can control the operation of the delivery member 78 and each of the stations of the machine 72 described above. For instance, the dosing station 36 can include any suitable apparatus or sensor as described above to identify when one of the substrates 23 has traveled into alignment with the dosing head 46, and when no substrates 23 are aligned with the dosing head 46 that require delivery of liquid 25, and communicate the alignment information to the processor. The processor then controls operation of the dosing heads 48.

Once the substrates 23 have been dosed with the active pharmaceutical ingredient-containing liquid 25, the substrates 23 travel along the delivery member 78 to the third location whereby they are delivered to the conveyor 38. In this regard, the delivery member 78 can be disposed in a spatial relationship with respect to the conveyor, such that the substrates 23 can travel from the delivery member 78 to the conveyor 38. In one example, the delivery member 78 is supported by a delivery support member 82 of the dosing machine 72. Thus, the support structure 76 can include a base 77 that supports the conveyor 38 and packaging station 42, and the delivery support member 82 that supports the delivery member 78 in addition to the dosing station 36, at least one holding tank 26, the at least one hopper 76, and the post-processing station 40. The substrates 23 can be dried as they travel from the second location to the third location. Thus, the dosing machine 72 can include a post-processing station 40 between the second location and the third location. The post-processing station can be configured as described above. Thus, once the substrate 23 is processed during the processing step, the API can adhere to the substrate 23.

The delivery support member 82 can support the delivery member 78 at a location above the conveyor 38, such that the dosed substrates 23 can travel from the support structure 78 down toward the conveyor 38. In one example, the dosed substrates 23 can travel under gravitational forces from the support structure 78 toward the conveyor 38. For instance, the delivery member 78 can transport the substrates 23 to the third location defined by an opening 83 in the delivery support member 82. Thus, the substrates 23 can travel through the opening 83 toward the conveyor 38. Alternatively, the third location can be configured as a conveyor or other suitable transport member that is configured to transport the substrates 23 toward the conveyor. The third location can be configured as a single opening or conveyor that receives the dosed substrates 23 from all paths 81 or a plurality of the paths 81. Alternatively, each path 81 can have its own dedicated third location.

The dosing machine 72 can include packaging station 42 that delivers a plurality of packages 86 to the conveyor 38. In one example, the packages 86 are placed onto the conveyor 38 upstream of the third location. The dosing machine 72 can include a reservoir that contains a plurality of packages, and can further deliver the packages sequentially onto the conveyor 38. Alternatively, a separate machine can deliver the packages to the conveyor 38. The conveyor 38 causes the packages 86 to move to a position in alignment with a respective one of the third locations. Thus, at least one of the substrates 23 that travels from the delivery member 78 toward the conveyor 38 is delivered into a respective package 86. It is contemplated that in some examples, a single dosed substrate, such as a baked good, will be delivered into each package 86. In other examples, it is contemplated that a plurality of dosed substrates, such as dried fruits and/or nuts, will be delivered into each package 82. For instance, a plurality of the substrates 23 can be delivered from a plurality of the paths 81 up to all of the paths 81 to a single respective container 82. Alternatively, one or more conveyors 38 can deliver the packages to respective locations whereby the packages 82 receive the respective at least one substrate 23 from the respective one of the plurality of paths 81 via the dedicated third location. Thus, packages 86 can thus simultaneously receive the respective at least one substrate 23.

The packaging station 42 can further include a sealing station 88 that is configured to enclose the packages 86 after they have received the respective at least one substrate 23. In particular, the conveyor 38 delivers the packages 86 to the sealing station 88 after they have received the respective at least one substrate 23. The sealing station can seal the package 86 and cause the package 86 to adhere to itself, for instance if the package 86 is a plastic bag or alternatively configured plastic package. The sealing station 88 can alternatively deliver and tighten a cap onto the package 86 if, for instance, the package is configured as a jar or other suitably configured package. The sealed package can then be delivered to a customer.

It should be noted that the illustrations and discussions of the embodiments and examples shown in the figures are for exemplary purposes only and should not be construed limiting the disclosure. One skilled in the art will appreciate that the present disclosure contemplates a range of possible modifications of the various aspects, embodiments and examples described herein. Additionally, it should be understood that the concepts described above with the above-described embodiments and examples may be employed alone or in combination with any of the other embodiments and examples described above. It should further be appreciated that the various alternatives described above with respect to one illustrated embodiment can apply to all other embodiments and examples described herein, unless otherwise indicated. Reference is therefore made to the claims.

In the following, further embodiments are described to facilitate the understanding of the invention:
1. A method of delivering an active pharmaceutical ingredient (API) to a substrate, the method comprising the steps of:
   bringing the substrate into alignment with a dosing station that includes a dosing head; and
   after the bringing step, delivering at least one microdroplet of an API-containing liquid from the dosing head to the substrate, wherein the at least one microdroplet contains a predetermined approximate quantity of API.
2. The method of embodiment 1, wherein the at least one microdroplet comprises a plurality of microdroplets, and the delivering step comprises delivering a plurality of microdroplets from the dosing head onto the substrate.
3. The method of any one of the preceding embodiments, wherein the dosing station comprises an array of dosing heads, and the delivering step comprises delivering a plurality of microdroplets from the array of dosing heads onto the substrate.
4. The method of embodiment 3, wherein a first one of the dosing heads delivers a first API, and a second one of the dosing heads delivers a second API different than the first API.
5. The method of any one of embodiments 1 to 3, wherein the API-containing liquid comprises a solution of the API and a solvent.
6. The method of embodiment 5, wherein the API has a concentration that is in a range from approximately 50 micrograms per microliter of solution to approximately 1 milligram per microliter of solution.
7. The method of any one of the preceding embodiments, wherein the microdroplet contains a microquantity of API in a range from approximately 0.1 micrograms to approximately 10 milligrams.
8. The method of any one of the preceding embodiments, wherein the microdroplet has a volume that is in a range from approximately 2 nanoliters to approximately 10 microliters.
9. The method of embodiment 8, wherein the volume is in a range from 25 nanoliters to approximately 2 microliters.
10. The method of embodiment 9, wherein the volume is in a range from approximately 50 nanoliters to approximately 1 microliter.
11. The method of any one of embodiments 8 to 10, wherein the volume is within a range from approximately 1% to approximately 5%, from a predetermined target volume of microdroplets at three sigma.
12. The method of any one of the preceding embodiments, wherein the substrate comprises an edible product.
13. The method of embodiment 12, wherein the edible product is cooked prior to the bringing step.
14. The method of embodiment 13, further comprising the step of cooking the edible product on a first side of a jurisdictional boundary, transporting the cooked edible product to a second side of a jurisdictional boundary, and performing the bringing step on the second side of the jurisdictional boundary.
15. The method of any one of embodiments 12 to 14, wherein the edible product is a dried fruit or nut.
16. The method of any one of embodiments 12 to 14, wherein the edible product is a gummy candy.
17. The method of any one of the preceding embodiments, wherein the bringing step comprises bringing a plurality of substrates into alignment with the dosing station, and the delivering step comprises delivering at least one microdroplet of API-containing liquid to each of the plurality of substrates.
18. The method of any one of embodiments 1 to 16, wherein the bringing step comprises bringing a plurality of substrates into alignment with respective different dosing stations, and the dosing stations deliver respective APIs to the aligned substrates, wherein the respective APIs have at least one API characteristic different from each other.
19. The method of any one of embodiments 1 to 16, wherein the bringing step comprises bringing a plurality of substrates into alignment with the dosing station, and the dosing station delivers respective APIs to different ones of the aligned substrates, wherein the respective APIs have at least one API characteristic different from each other.
20. The method of any one of embodiments 18 to 19, wherein the different API characteristic comprises at least one of 1) a concentration of the API, 2) a volume of API delivered to the substrates during the delivering step, 3) a composition of the API, and 4) a modifier mixed with the API, the modifier configured to modify at least one of a flavor, a mechanical property, and an aesthetic of the delivered API, and 5) a location of at least one dosing zone of the substrates.
21. The method of any one of embodiments 1 to 3, wherein the API-containing liquid comprises a pure API.
22. The method of any one of the preceding embodiments, wherein the API comprises a cannabinoid.
23. The method of any one of the preceding embodiments, wherein the delivering step comprises delivering a different API to different subzones in a delivery zone of the substrate.
24. The method of any one of the preceding embodiments, wherein the bringing step comprises causing the substrate to travel from a hopper to a first location of a delivery member, move along the delivery member from the first location to a second location, performing the delivery step at the second location, and subsequently moving the substrate from the second location to a third location whereby the substrate is transported to a packaging station, wherein the delivery member, the dosing station, and the packaging station are included in a stand-alone unitary dosing machine supported by a common support structure of the machine.
25. The method of any one of the preceding embodiments, further comprising, after the delivery step, post-processing the substrate, wherein post-processing at least one of 1) dries a solvent, 2) increases a viscosity of the API, 3) further adheres the API to the substrate, 4) disperses the API along the substrate, and 5) increases absorption of the API into the substrate.
26. A system for delivering an active pharmaceutical ingredient to a prepared substrate, the system comprising:
   a delivery station that includes a support surface configured to receive the prepared substrate; and
   a dosing station that is configured to deliver to the substrate a predetermined approximate dose of the active pharmaceutical ingredient,
   wherein at least one of the support surface and the dosing station is movable with respect to the other of the support surface and the dosing station so as to align the prepared substrate with the dosing station after the support surface has received the prepared substrate.
27. The system of embodiment 26, wherein the dosing station comprises at least one dosing head configured to receive a respective quantity of the active pharmaceutical ingredient, and deliver the respective quantity to the edible product.
28. The system of embodiment 27, wherein the at least one dosing head comprises a plurality of dosing heads.
29. The system of embodiment 28, wherein the respective quantity of the active pharmaceutical ingredient delivered by each of the dosing heads is substantially equal to the respective quantity of the active pharmaceutical ingredient delivered by the other dosing heads of the plurality of dosing heads.
30. The system of embodiment 28, wherein the respective quantity of the active pharmaceutical ingredient delivered by each of the dosing heads is different than to the respective quantity of the active pharmaceutical ingredient delivered by the other dosing heads of the plurality of dosing heads.
31. The system of any one of embodiments 28 to 30, wherein the quantity of each of the dosing heads cumulatively substantially defines the predetermined dose of the active pharmaceutical ingredient.
32. The system of any one of embodiments 28 to 31, wherein the dosing heads are positioned to deliver their respective quantities to different respective locations in a dosing zone of the edible product.
33. The system of any one of embodiments 28 to 32, configured to deactivate select dosing heads of the plurality of dosing heads that are out of alignment with the dosing zone.
34. The system of any one of embodiments 28 to 33, configured to activate select dosing heads of the plurality of dosing heads that are aligned with the dosing zone.
35. The system of any one of embodiments 31 to 34, further comprising a sensor to identify the dosing zone of the edible product, and each different location is disposed in the dosing zone.
36. The system of embodiment 35, wherein the dosing zone is at least partially defined by an outer perimeter of the edible product.
37. The system of embodiment 36, wherein the dosing zone is defined entirely by the outer perimeter of the edible product.
38. The system of any one of embodiments 35 to 36, wherein the dosing zone has a substantially predetermined location with respect to the outer perimeter of the edible product.
39. The system of any one of embodiments 26 to 38, wherein the active pharmaceutical ingredient is delivered to the substrate as a powder at the dosing station.
40. The system of any one of embodiments 26 to 39, further comprising a post-processing station that is configured to cause the active pharmaceutical ingredient to adhere to the substrate.
41. The system of embodiment 40, wherein the post-processing station is configured to direct at least one of light, and a forced gas to the active pharmaceutical ingredient.
42. The system of embodiment 41, wherein the forced gas is heated.
43. The system of embodiment 41, wherein the forced gas is substantially unheated.
44. The system of embodiment 41, wherein the forced gas is cooled.
45. The system of any one of embodiments 26 to 38, wherein the active pharmaceutical ingredient is delivered to the substrate as a liquid at the dosing station.
46. The system of embodiment 45, further comprising at least one holding tank that is configured to retain the liquid, wherein the at least one holding tank is in fluid communication with the dosing station.
47. The system of embodiment 46, wherein the liquid is an extract of the active pharmaceutical ingredient.
48. The system of embodiment 46, wherein liquid comprises a solution containing an approximate concentration of the active pharmaceutical ingredient.
49. The system of embodiment 48, wherein the active pharmaceutical ingredient defines a solute of a solution, and the active pharmaceutical ingredient defines a solvent that is configured to be evaporated.
50. The system of embodiment 49, wherein the solvent comprises an alcohol.
51. The system of any one of embodiments 27 to 50, wherein each of the dosing heads is configured to deliver microdroplets of the respective quantity of solution to the respective location.
52. The system of embodiment 51, wherein the dosing heads are spaced above the support surface along a direction of travel, and the microdroplets have a maximum cross-sectional dimension along a direction perpendicular to the direction of travel, the maximum cross-sectional dimension being is in a range from approximately 5 millionths of an inch to approximately 100 thousandths of an inch.
53. The system of embodiment 52, wherein the range is from approximately 5 thousandths of an inch to approximately 50 thousandths of an inch.
54. The system of embodiment 53, wherein the range is from approximately 20 thousandths of an inch to approximately 40 thousandths of an inch.
55. The system of any one of embodiments 52 to 54, wherein at least some of the microdroplets are substantially spherical shaped.
56. The system of any one of embodiments 52 to 54, wherein at least some of the microdroplets are teardrop shaped.
57. The system of any one of embodiments 51 to 56, wherein the microdroplets are delivered to an outer surface of the edible product.
58. The system of embodiment 57, wherein the microdroplets substantially remain on the outer surface of the edible product.
59. The system of embodiment 57, wherein the microdroplets permeate through the surface of the edible product so as to impregnate at least a volume of a thickness of the edible product that extends from the outer surface and an opposed inner surface that is supported by the support surface.
60. The system of any one of the embodiments 48 to 59, further comprising at least one conduit that is configured to deliver the solution from the at least one tank, respectively, toward the plurality of dosing heads, and a heater that is configured to deliver heat to the solution in the at least one conduit.
61. The system of any one of embodiments 45 to 60, further comprising an injection reservoir disposed between the holding tank and the dosing heads, and configured to receive the solution from the holding tank, the injection reservoir configured to deliver the solution to the dosing heads under positive pressure.
62. The system of any one of embodiments 46 to 61, further comprising a conveyor that defines the support surface, wherein the conveyor is configured to deliver the support surface from the delivery station to the dosing station.
63. The system of embodiment 62, wherein the conveyor defines a plurality of support surfaces, each configured to receive a respective edible product and deliver the edible product from the delivery station to the dosing station.
64. The system of embodiment 63, wherein the conveyor comprises a plurality of pockets that define the plurality of support surfaces, respectively.
65. The system of any one of embodiments 45 to 47, further comprising a post-processing station that is configured to cause the active pharmaceutical ingredient to adhere to the substrate.
66. The system of embodiment 65, wherein the post-processing station is configured to deliver a drying agent to the delivered liquid.
67. The system of embodiment 66, wherein the drying agent comprises at least one of light and a forced gas.
68. The system of embodiment 67, wherein the forced gas is heated.
69. The system of embodiment 67, wherein the forced gas is cooled.
70. The system of embodiment 67, wherein the forced gas is substantially unheated.
71. The system of any one of embodiments 48 to 64, further comprising a post-processing station that is configured to cause the active pharmaceutical ingredient to adhere to the substrate.
72. The system of embodiment 71, wherein the post-processing station is configured to deliver a drying agent to the delivered liquid.
73. The system of embodiment 72, wherein the drying agent comprises at least one of light and a forced gas.
74. The system of embodiment 73, wherein the forced gas is heated.
75. The system of embodiment 73, wherein the forced gas is substantially unheated.
76. The system of embodiment 73, wherein the forced gas is cooled.
77. The system of any one of embodiments 26 to 76, wherein the delivered active pharmaceutical ingredient is substantially evenly distributed on the substrate.
78. The system of embodiment 77, wherein the delivered active pharmaceutical ingredient is substantially evenly distributed along the substrate along two perpendicular directions.
79. The system of any one of embodiments 26 to 78, wherein the substrate is an edible product.
80. The system of any one of embodiments 26 to 78, wherein the substrate comprises an inedible product.
81. The system of any one of embodiments 26 to 79, wherein the edible product is a cooked edible food product.
82. The system of any one of embodiments 26 to 79, wherein the edible product is a raw edible food product.
83. The system of any one of embodiments 26 to 82, wherein the active pharmaceutical ingredient comprises at least one cannabinoid.
84. An active-containing edible product comprising:
   an edible product; and
   an active pharmaceutical ingredient carried by the edible product in a dosing zone in which the active pharmaceutical ingredient is substantially evenly distributed in the dosing zone along at least one direction.
85. The active-containing edible of embodiment 84, wherein the edible product is cooked.
86. The active-containing edible product of any one of embodiments 84 to 85, wherein the dosing zone defines a footprint that is greater than 75% of a footprint defined by an outer perimeter of the edible product.
87. The active-containing edible product of any one of embodiments 84 to 86, wherein the active pharmaceutical ingredient is not cooked.
88. The active-containing edible product of any one of embodiments 84 to 87, wherein at least a volume of the active pharmaceutical ingredient is disposed substantially at an outer surface of cooked edible product.
89. The active-containing edible product of any one of embodiments 84 to 88, wherein the outer surface of the baked good is opposite an inner surface of the baked good that is designed to rest against a support surface, the baked good defines a thickness from the inner surface to the outer surface, and at least 20% of the active pharmaceutical ingredient is disposed in a middle 75% of the thickness.
90. The active-containing edible product of embodiment 89, wherein at least 20% of the active pharmaceutical ingredient is disposed in a middle 50% of the thickness.
91. The active-containing edible product of any one of embodiments 84 to 90, wherein the active pharmaceutical ingredient comprises cannabis or hemp-derived material.
92. An active-containing substrate comprising:
   a substrate; and
   an active pharmaceutical ingredient carried by the substrate in a dosing zone in which the active pharmaceutical ingredient is substantially evenly distributed in the dosing zone along an outer surface of the substrate along at least one direction, and the distribution along the outer surface is different than a distribution of the active pharmaceutical ingredient along a thickness of the substrate.
93. The edible product of embodiment 92, wherein the dosing zone defines a footprint that is greater than half of a footprint defined by a maximum outer perimeter of the edible product.
94. The edible product of any one of embodiments 92 to 93, wherein the active pharmaceutical ingredient is not cooked.
95. The edible product of any one of embodiments 92 to 94, wherein the active pharmaceutical ingredient comprises at least one cannabinoid.
96. A method for delivering an active pharmaceutical ingredient to a substrate, the method comprising the steps of:
   storing a liquid in a holding tank, the liquid containing an approximate concentration of the active pharmaceutical ingredient; and
   delivering a microdroplet containing a predetermined volume of the liquid from the holding tank to the substrate at a dosing station, wherein the delivered predetermined quantity substantially contains a predetermined dose of the active pharmaceutical ingredient.
97. The method of embodiment 96, further comprising the step of delivering the substrate to a support surface at a delivery station, and moving at least one of the support surface and the delivery station with respect to the other of the support surface and the delivery station so as to bring the dosing station into alignment with the substrate.
98. The method of embodiment 97, wherein the support surface comprises a conveyor, the method comprising the step of moving the conveyor so as to correspondingly move the substrate from the delivery station to the dosing station.
99. The method of embodiment 98, wherein the conveyor comprises a plurality of pockets that define a plurality of support surfaces that are configured to receive a respective plurality of substrates and move the substrates to the dosing station.
100. The method of any one of embodiments 96 to 99, wherein the delivering step comprises delivering respective quantities of the volume of the liquid from a plurality of dosing heads to the substrate.
101. The method of embodiment 100, wherein the respective quantity of each of the dosing heads is substantially equal to the respective quantity of the other dosing heads of the plurality of dosing heads.
102. The method of embodiment 100, wherein the respective quantity of at least one of the dosing heads is different than the respective quantity of at least one other one of the other dosing heads of the plurality of dosing heads.
103. The method of any one of embodiments 100 to 102, wherein a first least one of the dosing heads is configured to deliver a first at least one active pharmaceutical ingredient to the substrate, and a second at least one of the dosing heads is configured to deliver a second at least one active pharmaceutical ingredient to the substrate.
104. The method of embodiment 103, further comprising the step of delivering the first and second at least one active pharmaceutical ingredient to respective different locations of the substrate.
105. The method of embodiment 103, further comprising the step of delivering the first and second at least one active pharmaceutical ingredient to the same location of the substrate.
106. The method of any one of embodiments 104 to 105, wherein the first and second at least one active pharmaceutical ingredients are delivered from a single dosing head.
107. The method of any one of embodiments 104 to 105, wherein the first and second at least one active pharmaceutical ingredients are delivered from respective first and second dosing heads.
108. The method of any one of embodiments 100 to 107, wherein the respective quantities of the dosing heads cumulatively substantially define the predetermined dose of the active pharmaceutical ingredient.
109. The method of any one of embodiments 100 to 108, wherein the delivering step comprises delivering the respective quantities from the dosing heads to respective locations of the substrate in a dosing zone of the substrate.
110. The method of embodiment 109, wherein the delivering step comprises delivering a plurality of microdroplets of the liquid to the respective locations.
111. The method of embodiment 110, wherein the dosing heads are spaced above the support surface along a vertical direction, and the microdroplets have a maximum cross-sectional dimension along a horizontal direction that is in a range from approximately 5 millionths of an inch to approximately 100 thousandths of an inch.
112. The method of embodiment 111, wherein the range is from approximately 5 thousandths of an inch to approximately 50 thousandths of an inch.
113. The method of embodiment 112, wherein at least some of the microdroplets are substantially spherical shaped.
114. The method of any one of embodiments 111 to 113, wherein at least some of the microdroplets are teardrop shaped.
115. The method of any one of embodiments 110 to 114, wherein the delivering step comprises delivering the microdroplets to an outer surface of the substrate.
116. The method of embodiment 115, wherein the delivering step causes the microdroplets to substantially remain on the outer surface of the substrate.
117. The method of embodiment 116, wherein the delivering step causes the microdroplets to permeate through the surface of the substrate so as to impregnate at least a volume of a thickness of the substrate that extends from the outer surface and an opposed inner surface that is supported by the support surface.
118. The method of any one of embodiments 96 to 109, wherein the delivering step comprises the step of injecting at least some of the volume of the liquid into the substrate.
119. The method of embodiment 118, wherein the delivering step comprises the step of injecting an entirety of the volume of the liquid into the substrate.
120. The method of any one of the embodiments 100 to 119, further comprising the step of delivering the volume of the liquid from the tank toward the plurality of dosing heads.
121. The method of embodiment 120, further comprising the step of heating the volume of the liquid as it travels from the tank toward the plurality of dosing heads.
122. The method of any one of embodiments 100 to 119, further comprising the step of delivering the volume of the liquid to an injection reservoir disposed between the holding tank and the dosing heads, and delivering the volume of the liquid from the injection reservoir to the dosing heads under positive pressure.
123. The method of any one of embodiments 100 to 122, further comprising the step of adjusting an internal volume of the dosing heads that defines the respective quantity to be delivered to the substrate.
124. The method of any one of embodiments 100 to 123, further comprising the step of deactivating select dosing heads of the plurality of dosing heads that are out of alignment with the dosing zone.
125. The method of any one of embodiments 100 to 123, further comprising the step of activating select dosing heads of the plurality of dosing heads that are aligned with the dosing zone.
126. The method of any one of embodiments 109 to 125, further comprising the step of sensing the dosing zone, such that each different location is disposed in the dosing zone.
127. The method of any one of embodiments 96 to 126, wherein the liquid is an extract, and the method further comprises the step of decreasing a viscosity of the liquid.
128. The method of any one of embodiments 126 to 127, further comprising the step of causing the active pharmaceutical ingredient to adhere to the substrate.
129. The method of embodiment 128, further comprising the step of moving the substrate from the dosing station to a post-processing station that comprises a plurality of drying heads configured to deliver a drying agent to the respective locations of the substrate.
130. The method of embodiment 129, wherein the drying agent comprises at least one of light and a forced gas.
131. The method of embodiment 129, wherein the forced gas is heated.
132. The method of embodiment 129, wherein the forced gas is substantially unheated.
133. The method of embodiment 129, wherein the forced gas is cooled.
134. The method of any one of embodiments 96 to 126, wherein the liquid comprises a solution containing the active pharmaceutical ingredient and a solvent, or a pure active pharmaceutical ingredient.
135. The method of embodiment 134, further comprising the step of causing the solvent to evaporate from an outer surface of the substrate.
136. The method of embodiment 134, further comprising the step of moving the substrate from the dosing station to a post-processing station that comprises a plurality of drying heads configured to deliver a drying agent to the respective locations of the substrate.
137. The method of embodiment 136, wherein the drying agent comprises at least one of light and a forced gas.
138. The method of embodiment 137, wherein the forced gas is heated.
139. The method of embodiment 137, wherein the forced gas is substantially unheated.
140. The method of embodiment 137, wherein the forced gas is cooled.
141. The method of any one of embodiments 135 to 140, further comprising the step of causing the active pharmaceutical ingredient to adhere to the substrate.
142. The method of any one of embodiments 96 to 141, wherein the substrate is an edible product.
143. The method of any one of embodiments 96 to 142, wherein the substrate comprises an inedible product.
144. The method of any one of embodiments 96 to 142, wherein the edible product is a cooked edible food product.
145. The method of embodiment 144, wherein the cooked edible product is a baked edible food product.
146. The system of embodiment 143, wherein the edible product is a raw edible food product.
147. The method of any one of embodiments 96 to 146, wherein the active pharmaceutical ingredient comprises at least one cannabinoid.
148. A method for delivering an active pharmaceutical ingredient to a substrate, the method comprising the steps of:
   delivering a predetermined mass of powder containing the active pharmaceutical ingredient to the substrate at a dosing station, wherein the delivered predetermined mass substantially contains a predetermined dose of the active pharmaceutical ingredient.
149. The method of embodiment 148, further comprising the step of delivering the substrate to a support surface at a delivery station, and moving at least one of the support surface and the delivery station with respect to the other of the support surface and the delivery station so as to bring the dosing station into alignment with the substrate.
150. The method of embodiment 149, wherein the support surface is defined by a pocket in a conveyor, the method comprising the step of moving the conveyor so as to correspondingly move the substrate from the delivery station to the dosing station.
151. The method of embodiment 150, wherein the conveyor comprises a plurality of pockets that define a plurality of support surfaces that are configured to receive a respective plurality of substrates and move the substrates to the dosing station.
152. The method of any one of embodiments 148 to 151, wherein the delivering step comprises delivering respective quantities of the powder from a plurality of dosing heads to the substrate.
153. The method of embodiment 152, wherein the respective quantity of each of the dosing heads is substantially equal to the respective quantity of the other dosing heads of the plurality of dosing heads.
154. The method of embodiment 152, wherein the respective quantity of at least one of the dosing heads is different than the respective quantity of at least one other one of the other dosing heads of the plurality of dosing heads.
155. The method of any one of embodiments 152 to 154, wherein a first least one of the dosing heads is configured to deliver a first at least one active pharmaceutical ingredient to the substrate, and a second at least one of the dosing heads is configured to deliver a second at least one active pharmaceutical ingredient to the substrate.
156. The method of embodiment 155, further comprising the step of delivering the first and second at least one active pharmaceutical ingredient to respective different locations of the substrate.
157. The method of embodiment 155, further comprising the step of delivering the first and second at least one active pharmaceutical ingredient to the same location of the substrate.
158. The method of any one of embodiments 156 to 157, wherein the first and second at least one active pharmaceutical ingredients are delivered from a single dosing head.
159. The method of any one of embodiments 156 to 157, wherein the first and second at least one active pharmaceutical ingredients are delivered from respective first and second dosing heads.
160. The method of any one of embodiments 152 to 159, wherein the respective quantities of the dosing heads cumulatively substantially define the predetermined dose of the active pharmaceutical ingredient.
161. The method of any one of embodiments 152 to 160, wherein the delivering step comprises delivering the respective quantities from the dosing heads to respective locations of the substrate in a dosing zone of the substrate.
162. The method of any one of embodiments 152 to 161, further comprising the step of deactivating select dosing heads of the plurality of dosing heads that are out of alignment with the dosing zone.
163. The method of any one of embodiments 152 to 161, further comprising the step of activating select dosing heads of the plurality of dosing heads that are aligned with the dosing zone.
164. The method of any one of embodiments 161 to 163, further comprising the step of sensing the dosing zone, such that each different location is disposed in the dosing zone.
165. The method of any one of embodiments 148 to 164, further comprising the step of causing the active pharmaceutical ingredient to adhere to the substrate.
166. The method of embodiment 165, further comprising the step of moving the substrate from the dosing station to a post-processing station that comprises a plurality of drying heads configured to deliver a drying agent to the respective locations of the substrate, wherein the drying agent causes the pharmaceutical ingredient to adhere to the substrate.
167. The method of embodiment 166, wherein the drying agent comprises at least one of light and a forced gas.
168. The method of embodiment 167, wherein the forced gas is heated.
169. The method of embodiment 167, wherein the forced gas is substantially unheated.
170. The method of embodiment 167, wherein the forced gas is cooled.
171. The system of any one of embodiments 148 to 170, wherein the substrate is an inedible product.
172. The system of any one of embodiments 148 to 170, wherein the substrate is an edible product.
173. The system of embodiment 172, wherein the edible product is a cooked edible food product.
174. The method of embodiment 173, wherein the cooked edible product is a baked edible food product.
175. The system of embodiment 172, wherein the edible product is a raw edible food product.
176. The method of any one of embodiments 148 to 175, wherein the active pharmaceutical ingredient comprises at least one cannabinoid.
177. A method for transforming an edible product into a cannabis-containing edible product, comprising:
   an edible product;
   a cannabis or hemp-derived material; and
   an applicator;
   wherein the applicator is configured to add cannabis or hemp-derived material to the edible product.
178. The method of embodiment 177, wherein the applicator sprays cannabis or hemp-derived material onto a surface of the edible product; wherein the cannabis or hemp-derived material either diffuses into or remains on the surface of the edible product.
179. The method of embodiment 177, wherein the applicator shoots cannabis or hemp-derived material into the edible product.
180. The method of embodiment 177, wherein the applicator squirts cannabis or hemp-derived material into the edible product.
181. The method of embodiment 180, wherein a heated applicator is used to squirt the cannabis or hemp-derived material into the edible product.
182. The method of embodiment 177, wherein the applicator squirts cannabis or hemp-derived material onto a surface of the edible product.
183. The method of embodiment 177, wherein the applicator stamps cannabis or hemp-derived material onto a surface of the edible product.
184. The method of embodiment 177, wherein the applicator prints cannabis or hemp-derived material onto a surface of the edible product.
185. The method of embodiment 177, wherein the applicator sprinkles cannabis or hemp-derived material onto a surface of the edible product.
186. The method of embodiment 177, wherein the applicator builds one or more micro-pills on a surface of the edible product.
187. The method of embodiment 177, wherein the applicator adds a conformal coating of the cannabis or hemp derived material to the edible product.
188. The method of embodiment 177, wherein the applicator encapsulates or mixes the cannabis or hemp derived material with at least one modifier that is configured to modify flavor, mechanical properties, or aesthetics of applied cannabis or hemp material before being delivered to the edible product.
189. The method of embodiment 177, wherein the applicator adjusts size, location, or distribution of the cannabis or hemp material on the edible product to modify flavor or aesthetics.
190. The method of embodiment 177, wherein the applicator adds energy for improved diffusion or post-application processing of the cannabis or hemp derived material.
191. A method for transforming an edible product into a cannabis edible, the method comprising the step of delivering a cannabis or hemp-derived material from an applicator to an edible product.
192. The method of embodiment 191, wherein the delivering step comprises spraying the cannabis or hemp-derived material onto an outer surface of the edible product.
193. The method of embodiment 192, wherein the spraying step causes the cannabis or hemp-derived material to remain on the outer surface of the edible product.
194. The method of any one of embodiments 192 to 193, wherein the spraying step causes the cannabis or hemp-derived material to either diffuse into the edible product.
195. The method of embodiment 191, wherein the delivering step comprises shooting the cannabis or hemp-derived material into the edible product.
196. The method of embodiment 191, wherein the delivering step comprises squirting the cannabis or hemp-derived material into the edible product.
197. The method of embodiment 191, wherein the delivering step comprises squirting the cannabis or hemp-derived material onto an outer surface of the edible product.
198. The method of embodiment 191, wherein the delivering step comprises stamping the cannabis or hemp-derived material onto an outer surface of the edible product.
199. The method of embodiment 191, wherein the delivering step comprises printing the cannabis or hemp-derived material onto an outer surface of the edible product.
200. The method of embodiment 191, wherein the delivering step comprises sprinkling cannabis or hemp-derived material onto an outer surface of the edible product.
201. The method of embodiment 191, wherein the delivering step comprises building one or more micro-pills of the cannabis or hemp-derived material on an outer surface of the edible product.
202. The method of embodiment 191, wherein the delivering step comprises adding a conformal coating of the cannabis or hemp derived material to the edible product.
203. The method of embodiment 191, further comprising, before the delivering step, mixing the cannabis or hemp derived material with at least one modifier so as to modify at least one of flavor, one or more mechanical properties, or one or more aesthetics of the cannabis or hemp material.
204. The method of embodiment 203, wherein the mixing step occurs in the applicator.
205. The method of any one of embodiments 191 to 204, further comprising the step of adjusting at least one of a size, location, or distribution of the cannabis or hemp material on the edible product to modify flavor or aesthetics.
206. The method of embodiment 205, wherein the adjusting step is performed in the applicator.
207. The method of any one of embodiments 191 to 206, wherein the applicator is heated, and the cannabis or hemp-derived material is heated during the delivering step.
208. The method of any one of embodiments 191 to 207, further comprising the step of adding energy to a surface of the edible so as to improve adhesion of the cannabis or hemp-derived material to the surface.
209. The method of embodiment 208, wherein the step of adding energy comprises increasing a temperature at the surface.
210. The method of embodiment 209, wherein the step of increasing temperature comprises directing at least one of forced air and a light to the surface.
211. The method of any one of embodiments 191 to 210, wherein the cannabis or hemp-derived material is a solute in a liquid solution.
212. The method of embodiment 211, wherein the solution includes a solvent.
213. The method of embodiment 211, wherein the solvent comprises an alcohol.
214. The method of any one of embodiments 211 to 213, further comprising the step of adjusting a concentration or ingredient of the solution.
215. The method of any one of embodiments 191 to 210, wherein the cannabis or hemp-derived material is pure.
216. A method of applying an active pharmaceutical ingredient to a quantity of fruits or nuts, the method comprising the steps of applying microdroplets to at least one of the quantity of fruits or nuts, the microdroplets containing a dosage of API.
217. A method for delivering an active pharmaceutical ingredient (API) to a quantity of at least one of fruits and nuts, the method comprising the steps of:
   storing an API containing material in a holding tank, the API containing material containing a known concentration of the active pharmaceutical ingredient; and
   delivering microquantities of the API containing material to the fruits or nuts, thereby controlling the dosage of the API on each of the fruits or nuts to within 10% of a predetermined dosage at three sigma.
218. The method of embodiment 217, wherein the API containing material comprises a liquid, and the microquanties comprise microdroplets of the API containing material.
219. The method of embodiment 218, wherein the liquid includes a concentration of the API and a solvent.
220. The method of embodiment 219, wherein the solvent comprises alcohol.
221. The method of any one of embodiments 219 to 220, wherein the liquid contains a concentration of the API that is in a range from approximately 40% to approximately 90% by volume.
222. The method of embodiment 221, wherein the liquid contains the concentration of the API is in a range from approximately 50% to approximately 70%.
223. The method of any one of embodiments 219 to 222, wherein the API comprises a cannabinoid.
224. The method of any one of embodiments 218 to 223, wherein the delivering step comprises delivering the API to the fruits or nuts along a direction of travel, and the microdroplets define a maximum cross-sectional dimension that is in a range from approximately 5 millionths of an inch to approximately 100 thousandths of an inch as measured along a direction perpendicular to the direction of travel.
225. The method of any one of embodiments 218 to 223, wherein the delivering step comprises delivering the API to the fruits or nuts along a direction of travel, and the microdroplets define a maximum cross-sectional dimension that is in a range from approximately 20 thousandths of an inch to approximately 40 thousandths of an inch as measured along a direction perpendicular to the direction of travel.
226. The method of any one of embodiments 218 to 225, wherein the delivering step comprises delivering the microdroplets from a dosing head at a distance from the fruits or nuts that is in a range from approximately 2mm to approximately 25 mm.
227. The method of any one of embodiments 218 to 226, wherein the microdroplets have respective volumes that range from approximately 2 nanoliters to approximately 10 microliters.
228. The method of embodiment 227, wherein the volume is in a range from approximately 25 nanoliters and approximately 2 microliters.
229. The method of embodiment 228, wherein the volume is in a range from approximately 50 nanoliters to approximately 1 microliter.
230. The method of any one of embodiments 218 to 229, wherein the microdroplets have a concentration of active pharmaceutical ingredient that is in a range from approximately 50 micrograms per microliter to approximately 1 milligram per microliter.
231. The method of any one of embodiments 217 to 230, wherein the dosage of API is in a range from approximately 0.5 micrograms to approximately 10 milligrams per individualized fruit or nut.
232. The method of embodiment 231, wherein the dosage of the API on each of the fruits or nuts is controlled to within 5% of the predetermined dosage at three sigma.
233. The method of embodiment 232, wherein the dosage of the API on each of the fruits or nuts is controlled to within approximately 1% of the predetermined dosage at three sigma
234. The method of any one of embodiments 217 to 233, wherein the quantity comprises fruits, and the fruits are raw.
235. The method of any one of embodiments 217 to 233, wherein the quantity comprises fruits, and the fruits are dried prior to the delivering step.
236. The method of any one of embodiments 217 to 233, wherein the quantity comprises fruits, and the fruits are candied prior to the delivering step.
237. The method of any one of embodiments 217 to 233, wherein the quantity comprises nuts, and the nuts are raw.
238. The method of any one of embodiments 217 to 233, wherein the quantity comprises nuts, and the nuts are cooked prior to the depositing step.
239. The method of any one of embodiments 237 to 238, wherein the nuts are salted prior to the depositing step.
240. The method of any one of embodiments 237 to 239, wherein the nuts are candied prior to the depositing step.
241. A quantity of fruits or nuts prepared by the method as recited in any one of embodiments 217 to 240.
242. A stand-alone dosing machine configured to deliver an active pharmaceutical ingredient (API) to at least one substrate, the system comprising:
   a delivery station configured to receive the at least one substrate;
   a dosing station configured to receive the substrate from the delivery station, and deliver to the at least one substrate an API-containing material;
   a conveyor configured to receive the at least one substrate from the dosing station, the conveyor configured to transport the at least one substrate to a packaging station,
   wherein the delivery station, the dosing station, the conveyor, and the packaging station are integrated into a single unitary dosing machine supported by a common support structure of the machine.
243. The stand-alone dosing machine of embodiment 242, wherein the API-containing material comprises a solvent, the machine further comprising a post-processing station configured evaporate the solvent.
244. The stand-alone dosing machine of any one of embodiments 242 to 243, wherein the dosing station comprises at least one dosing head configured to receive a microquantity of the API, and deliver the respective quantity to the edible product.
245. The stand-alone dosing machine of embodiment 244, wherein the at least one dosing head comprises an array of dosing heads.
246. The stand-alone dosing machine of embodiment 245, wherein the delivery station comprises a delivery member that receives the at least one substrate from a hopper at a first location, and delivers the at least one substrate to a second location that is in alignment with the dosing station, whereby the dosing station delivers a predetermined dose of the API to the at least one substrate.
247. The stand-alone dosing machine of embodiment 246, wherein dosing station comprises a plurality of dosing stations, and the delivery member defines a plurality of paths that each delivers respective paths of substrates to respective dosing stations of the machine.
248. The stand-alone dosing machine of any one of embodiments 246 to 247, wherein the delivery member comprises a plurality of grooves that defines the plurality of paths.
249. The stand-alone dosing machine of any one of embodiments 247 to 248, wherein the delivery member is configured to transport the at least one substrate from the first location to the second location.
250. The stand-alone dosing machine of embodiment 249, wherein the deliver member is sloped and configured to vibrate so as to cause the at least one substrate to move from the first location to the second location.
251. The stand-alone dosing machine of embodiment 249, wherein the deliver member is movable so as to move the substrate from the first location to the second location.
252. The stand-alone dosing machine of any one of embodiments 247 to 251, wherein the delivery member is configured to transport the at least one substrate from the second location to a third location whereby the at least one substrate is delivered to the conveyor.
253. The stand-alone dosing machine of embodiment 252, wherein the machine is programmed to deliver a predetermined quantity of substrates from the third location to a package disposed on the conveyor.
254. The stand-alone dosing machine of any one of embodiments 242 to 253, wherein the dosing station comprises at least one dosing head that is configured to deliver the API substantially evenly distributed along the substrate with respect to at least one direction.
255. The stand-alone dosing machine of embodiment 254, wherein the dosing station comprises an array of dosing heads each configured to deliver respective microquantities of the API to the substrate.
256. The stand-alone dosing machine of any one of embodiments 242 to 255, wherein the substrate is an edible product.
257. The stand-alone dosing machine of embodiment 256, wherein the substrate comprises at least one of a fruit and a nut.
258. The stand-alone dosing machine of embodiment 256, wherein the substrate comprises a baked good.
259. The stand-alone dosing machine of embodiment 256, wherein the substrate comprises a gummy candy.
260. The stand-alone dosing machine of any one of embodiments 242 to 255, wherein the substrate is an inedible product.
261. The stand-alone dosing machine of any one of embodiments 242 to 260, wherein the API comprises at least one cannabinoid.
262. The stand-alone dosing machine of any one of embodiments 242 to 261, wherein the API-containing material comprises a microdroplet.
263. The stand-alone dosing machine of embodiment 262, wherein the API-containing material comprises a pure API.
264. The stand-alone dosing machine of embodiment 262, wherein the API-containing material comprises a concentration of the API and a solvent.
265. The stand-alone dosing machine of embodiment 264, wherein the API is nonsolid.
266. The stand-alone dosing machine of any one of embodiments 264 to 265, wherein the solvent comprises alcohol.
267. The stand-alone dosing machine of any one of embodiments 265 to 266, wherein the liquid contains a concentration of the API that is in a range from approximately 40% to approximately 90% by volume.
268. The stand-alone dosing machine of embodiment 267, wherein the liquid contains the concentration of the API is in a range from approximately 50% to approximately 70%.
269. The stand-alone dosing machine of any one of embodiments 264 to 268, wherein the dosing station delivers the microdroplets to the substrate along a direction of travel, and the microdroplets define a maximum cross-sectional dimension that is in a range from approximately 5 millionths of an inch to approximately 100 thousandths of an inch as measured along a direction perpendicular to the direction of travel.
270. The stand-alone dosing machine of embodiment 269, wherein the maximum cross-sectional dimension that is in a range from approximately 20 thousandths of an inch to approximately 40 thousandths of an inch as measured along a direction perpendicular to the direction of travel.
271. The stand-alone dosing machine of any one of embodiments 264 to 270, wherein the microdroplets are delivered at a distance from the substrate that is in a range from approximately 2mm to approximately 25 mm.
272. The stand-alone dosing machine of any one of embodiments 264 to 271, wherein the microdroplets have respective volumes that range from approximately 2 nanoliters to approximately 10 microliters.
273. The stand-alone dosing machine of embodiment 272, wherein the volume is in a range from approximately 25 nanoliters and approximately 1 microliter.
274. The stand-alone dosing machine of embodiment 273, wherein the volume is in a range from approximately 50 nanoliters to approximately 2 microliters.
275. The stand-alone dosing machine of any one of embodiments 264 to 274, wherein the microdroplets have a concentration of active pharmaceutical ingredient that is in a range from approximately 50 micrograms per microliter to approximately 1 milligram per microliter.
276. The stand-alone dosing machine of any one of embodiments 264 to 275, wherein the substrate comprises a fruit or nut, and the API-containing material delivered to the fruit or nut includes a predetermined dosage of API that is in a range from approximately 0.5 micrograms to approximately 10 milligrams.
277. The stand-alone dosing machine of any one of embodiments 264 to 275, wherein the substrate comprises a gummy candy, and the API-containing material delivered to the gummy candy includes a predetermined dosage of API that is in a range from approximately 0.5 micrograms to approximately 10 milligrams.
278. The stand-alone dosing machine of any one of embodiments 265 to 277, wherein the range is from approximately 1 milligram to approximately 2 milligrams.
279. The stand-alone dosing machine of any one of embodiments 276 to 278, wherein the dosage is controlled to within 5% of the predetermined dosage at three sigma.
280. The stand-alone dosing machine of embodiment 279, wherein the dosage is controlled to within approximately 1% of the predetermined dosage at three sigma.
281. A method of dosing a food product with an active pharmaceutical ingredient, the method comprising the steps of:
   preparing a food product, on a first side of a jurisdictional boundary;
   transporting the prepared food product across the jurisdictional boundary to a second side of the jurisdictional boundary; and
   applying the active pharmaceutical ingredient to the transported food product.
282. The method of embodiment 281, wherein the food product is not dosed with the active pharmaceutical ingredient during the preparing step.
283. The method of any one of embodiments 281 to 282, wherein the food product is not dosed with the active pharmaceutical ingredient during the transporting step.
284. The method of any one of embodiments 281 to 283, wherein the active pharmaceutical ingredient comprises a cannabinoid.
285. The method of any one of embodiments 281 to 284, wherein the applying step comprises delivering at least one microdroplet of an active pharmaceutical agent-containing liquid to the prepared food product.
286. The method of any one of embodiments 281 to 285, wherein the preparing step comprises cooking the food product.

## Claims

1. A method of quantifying an active pharmaceutical ingredient delivered to a food product, the method comprising the steps of:
causing a plurality of microdroplets to travel out of a dosing head of a dosing system toward the food product, the microdroplets containing the active pharmaceutical ingredient that includes a cannabinoid;
measuring a cross-sectional dimension of each of the microdroplets with a camera as the microdroplets travel from the dosing head to the food product; and
based on the measuring step, calculating an approximation of a volume of the microdroplets delivered to the food product.

2. The method of claim 1, wherein each of the microdroplets has a respective volume in a range from approximately 2 nanoliters to approximately 10 microliters.

3. The method of claim 2, wherein the volume is in a range from approximately 25 nanoliters to approximately 2 microliters.

4. The method of claim 3, wherein the volume is in a range from approximately 25 nanoliters to approximately 1 microliter.

5. The method of any one of claims 1 to 4, further comprising the step of comparing the cross-sectional dimensions to each other to ensure consistency of the volume of the microdroplets delivered to the food product.

6. The method of any one of claims 2 to 5, further comprising the step of integrating the cross-sectional dimensions into a feedback loop associated with operation of the dosing system.

7. The method of any one of claims 1 to 6, wherein the microdroplets have an elongate shape when they leave the dosing head.

8. The method of claim 7, wherein the measuring step is performed after the microdroplets have transformed from the elongate shape into a shape that is more spherical than the elongate shape.

9. The method of claim 8, wherein the measuring step is performed after the microdroplets have transformed into an approximate spherical shape.

10. The method of any one of claims 1 to 9, wherein calculating step comprises the step of calculating an approximation of the volume of each of the microdroplets.

11. The method of claim 10, further comprising the step of comparing the approximations of the volumes of the microdroplets to each other to ensure consistency of the volume of the microdroplets delivered to the food product.

12. A dosing system for quantifying an active pharmaceutical ingredient delivered to a food product, the dosing system comprising:
a dosing head configured to deliver a plurality of microdroplets to the food product, wherein the microdroplets contain the active pharmaceutical ingredient that includes a cannabinoid;
a camera configured to measure a cross-sectional dimension of each of the microdroplets as the microdroplets travel from the dosing head to the food product,
wherein, the system is configured to calculate an approximation of a volume of the microdroplets delivered to the food product based on the measuring step measured cross-sectional dimension of each of the microdroplets.

13. The dosing system of claim 12, wherein each of the microdroplets has a respective volume in a range from approximately 2 nanoliters to approximately 10 microliters.

14. The dosing system of claim 13, wherein the volume is in a range from approximately 25 nanoliters to approximately 2 microliters.

15. The dosing system of claim 14, wherein the volume is in a range from approximately 25 nanoliters to approximately 1 microliter.

16. The dosing system of any one of claims 12 to 15, configured to compare the cross-sectional dimensions to each other to ensure consistency of the volume of the microdroplets delivered to the food product.

17. The dosing system of any one of claims 12 to 16, configured to integrate the measured cross-sectional dimensions into a feedback loop associated with operation of the dosing system.

18. The dosing system of any one of claims 12 to 17, wherein the microdroplets have an elongate shape when they leave the dosing head.

19. The dosing system of claim 18, wherein the camera is positioned to measure the cross-sectional dimension of each of the microdroplets after the microdroplets have transformed from the elongate shape into a shape that is more spherical than the elongate shape.

20. The dosing system of claim 19, wherein the camera is positioned to measure the cross-sectional dimension of each of the microdroplets after the microdroplets have transformed into an approximate spherical shape.

21. The dosing system of any one of claims 12 to 20, configured to calculate an approximation of the volume of each of the microdroplets.

22. The dosing system of claim 21, configured to compare the approximations of the volumes of the microdroplets to each other to ensure consistency of the volume of microdroplets delivered to the food product.
